# EUROPEAN PATENT APPLICATION

(11) **EP 4 671 251 A1**
(43) Date of publication of application: **31.12.2025**
(21) Application number: 24759781.8
(22) Date of filing: 23.02.2024
(51) Int. Cl.: C07D 498/22, A61K 31/438, A61P 31/04

(54) **SALTS OF COMPOUNDS, CRYSTAL FORMS THEREOF, PREPARATION METHOD THEREFOR AND USE THEREOF**

(30) Priority: 24.02.2023 CN 202310165445; 24.02.2023 CN 202310165662; 24.02.2023 CN 202310165722
(71) Applicant: TenNor Therapeutics (Suzhou) Limited, Suzhou, Jiangsu 215123 (CN)
(72) Inventor: LIU, Yu, Suzhou, Jiangsu 215123 (CN); YAO, Wenhui, Suzhou, Jiangsu 215123 (CN); ZHANG, Ling, Suzhou, Jiangsu 215123 (CN); WU, Zeqin, Suzhou, Jiangsu 215123 (CN); MA, Zhenkun, Suzhou, Jiangsu 215123 (CN)
(74) Representative: Weickmann & Weickmann PartmbB
(86) International application number: PCT/CN2024/078360
(87) International publication number: WO 2024/175107

(57) **Abstract**

Provided in the present application are salts of compounds, or hydrates, solvates, prodrugs, metabolites or deuterated compounds thereof, the salts being triphenylacetate salts of compounds of formula (I), L being a bond, an alkyl group or any alkoxy group in which a central carbon atom is substituted by an aryl group, and G being hydrogen, an alkyl group, a haloalkyl group, an alkenyl group, an alkynyl group, an optionally substituted cycloalkyl group, or an optionally substituted heterocyclyl group. Further provided in the present application are a plurality of crystal forms of the salts, a preparation method therefor, and the use thereof.

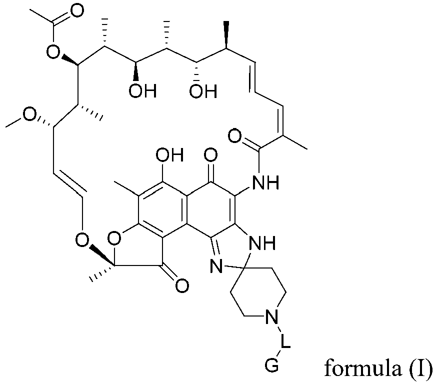

## Description

### TECHNICAL FIELD

The present application relates to the field of biopharmaceuticals, and in particular, to salts of compounds and crystal forms thereof, a preparation method therefor, and the use thereof.

### BACKGROUND

Rifamycins are natural products with potential antimicrobial activity. Rifamycin of natural sources (e.g., rifamycin B, rifamycin O, rifamycin R, rifamycin U, rifamycin S, rifamycin SV, and rifamycin Y (Brufani, M., Cerrini, S., Fedeli, W., Vaciago, A. J. Mol. Biol. 1974, 87, 409-435)) has limited therapeutic applications due to, for example, poor pharmacokinetics, low oral bioavailability, weak activity against gram-negative pathogens, and low distribution in infected tissues. In the prior art, various semisynthetic rifamycin derivatives with improved antibacterial spectrum and pharmacological properties have been produced through chemical modifications. Among such semisynthetic compounds, rifampicin and rifabutin, for example, have been developed as therapeutics currently used for the treatment of tuberculosis and other microbial infections (Farr, B. M. Rifamycins, in Principles and Practice ofInfectious Diseases; Mandell, G. L., Bennett, J. E., Dolin, R., Eds.; Churchhi 11 Livingstone Philadelphia; pp. 348-361). For another example, TNP-2198 is a dual-targeting rifamycin-nitroimidazole conjugate that has potent activity against microaerophilic and anaerobic bacterial pathogens and exhibits good activity against strains resistant to both rifamycin and nitroimidazole. However, the development and application of such innovative pharmaceutical molecules with antibacterial activity are often affected by such physicochemical properties as high relative molecular mass, high lipophilicity, low water solubility, hygroscopicity, and the like as well as bioavailability and stability (chemical stability, thermal stability, melting point, crystal form stability) of the free forms, making it is difficult to meet the requirements for druggability. In the art, some progress has also been made in the research on new drugs of rifamycin derivatives in recent years, such as crystal forms of rifabutin (e.g., CN103408571B). However, the experiments provided in the present application show that rifabutin still faces the problems of low purity and difficulties in meeting the druggability requirements. Therefore, the search and screening for new molecular entities (e.g., salt forms or crystal forms) of such parent drug molecules are crucial to their pharmaceutical applications.

### SUMMARY

In order to overcome the defects in the prior art, the present application provides salt forms of the compounds of formula (I) with better stability and solubility that can satisfy the pharmaceutical requirements, stable crystal forms of the salt forms, a preparation method therefor, and the use thereof. In one aspect, the present application provides a triphenylacetate salt of the compound of formula (I), or a hydrate, solvate, prodrug, metabolite or deuteride thereof: 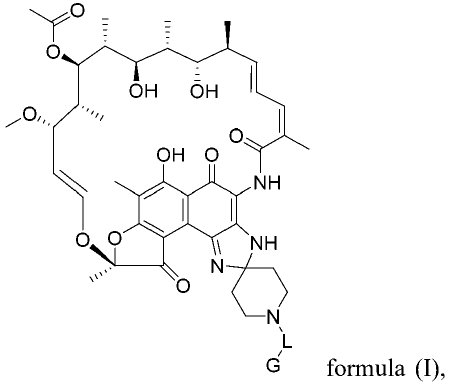 wherein L is a bond, alkyl, or the structure shown in 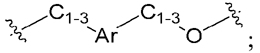 Ar is optionally substituted aryl; G is hydrogen, alkyl, haloalkyl, alkenyl, alkynyl, optionally substituted cycloalkyl, or optionally substituted heterocyclyl.

In some embodiments, the molar ratio of the compound of formula (I) to triphenylacetic acid is 0.8-1.2.

In some embodiments, the salt is a monotriphenylacetate salt.

In some embodiments, L is C₁-C₆ alkyl.

In some embodiments, the structure of L is:

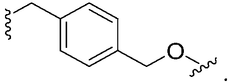

In some embodiments, G is C₁-C₆ alkyl, C₂-C₆ alkenyl, or C₂-C₆ alkynyl.

In some embodiments, G is C₁-C₃ monohaloalkyl or C₁-C₃ dihaloalkyl.

In some embodiments, G is optionally substituted C₃-C₈ cycloalkyl, optionally substituted 3- to 8-membered heteroatom-containing monocyclic substituent, or optionally substituted 4- to 12-membered heteroatom-containing bicyclic substituent.

In some embodiments, G is any one of the following structures:

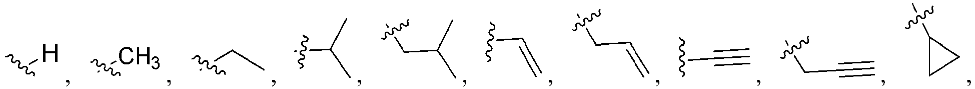

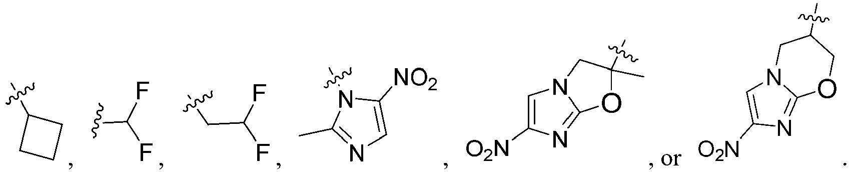

In some embodiments, the optionally substituted substituent is selected from: halogen, hydroxyl, carboxyl, carbonyl, amino, nitro, thiol, cyano, alkyl, alkenyl, alkynyl, cycloalkyl, aryl, heteroaryl, alkylamino, alkoxy, and carbonyl ester group.

In some embodiments, the structure of 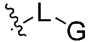 is any one of the following structures:

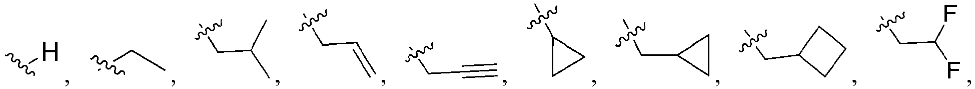

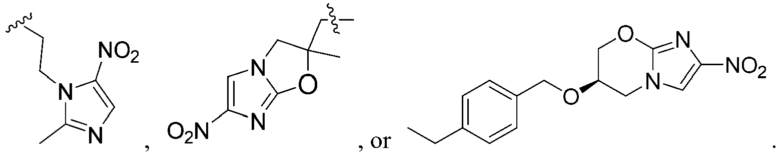

In some embodiments, the triphenylacetate salt is a crystal, and the crystal is crystal form I formed by crystallizing the salt of formula (I-I): 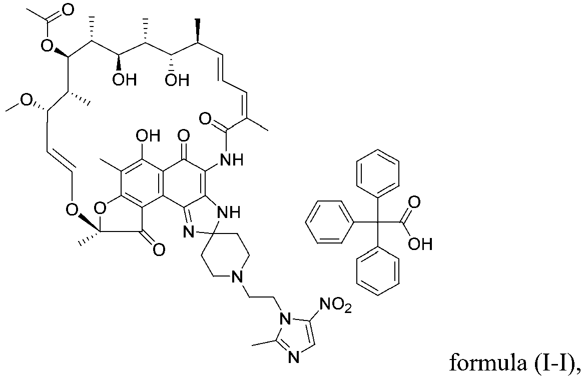 wherein the crystal I has an X-ray powder diffraction pattern comprising characteristic peaks at diffraction angles 20 of 6.11±0.2°, 9.93±0.2°, 11.49±0.2°, 14.41±0.2°, 16.15±0.2°, and 18.86±0.2°.

In some embodiments, the crystal form I has an X-ray powder diffraction pattern comprising characteristic peaks at one or more diffraction angles 20 of 12.46±0.2°, 14.89±0.2°, 16.81±0.2°, 19.31±0.2°, 20.34±0.2°, and 24.35±0.2°.

In some embodiments, the crystal form I has an X-ray powder diffraction pattern comprising characteristic peaks at diffraction angles 20 of 12.46±0.2°, 14.89±0.2°, 16.81±0.2°, 19.31±0.2°, 20.34±0.2°, and 24.35±0.2°.

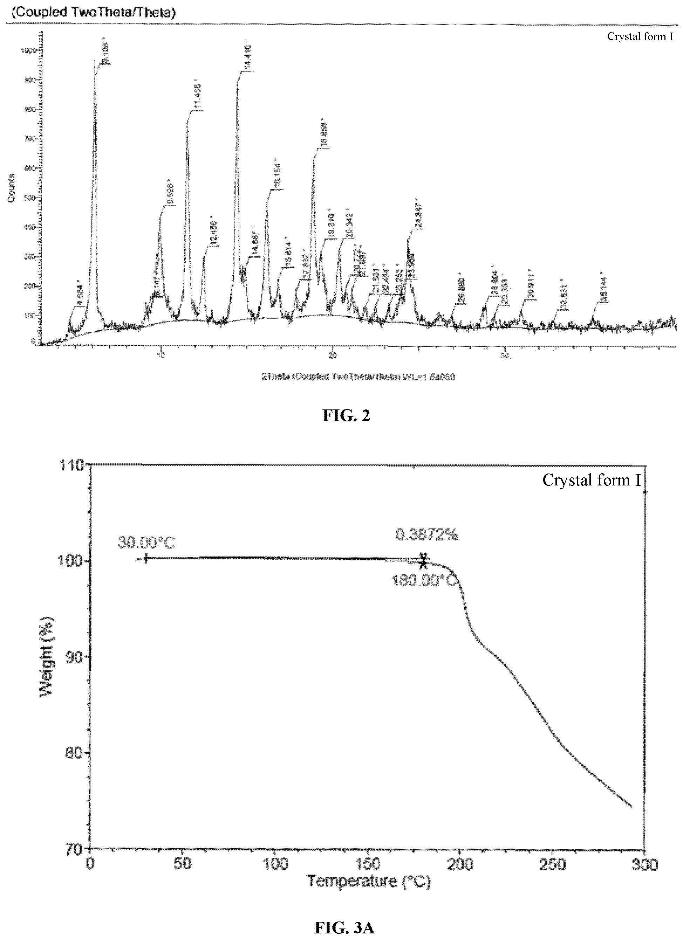

In some embodiments, the crystal form I has an X-ray powder diffraction pattern substantially consistent with FIG. 2.

In some embodiments, the crystal form I is an anhydrate.

In some embodiments, the crystal form I has a differential scanning calorimetry profile comprising an endothermic peak at 174.2 °C.

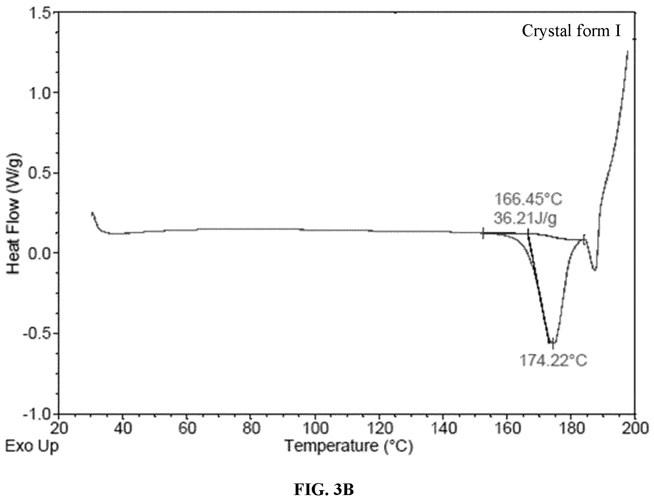

In some embodiments, the crystal form I has a differential scanning calorimetry profile substantially consistent with FIG. 3B.

In some embodiments, the triphenylacetate salt is a crystal, and the crystal is crystal form II formed by crystallizing the salt of formula (I-I): 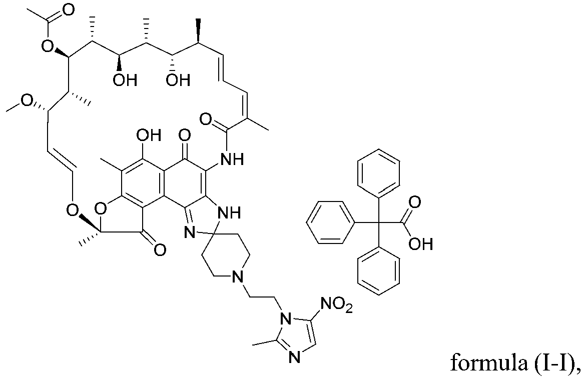 wherein the crystal II has an X-ray powder diffraction pattern comprising characteristic peaks at diffraction angles 2θ of 11.37±0.2°, 12.06±0.2°, 16.10±0.2°, 18.14±0.2°, 19.80±0.2°, and 24.36±0.2°.

In some embodiments, the crystal form II has an X-ray powder diffraction pattern comprising characteristic peaks at one or more diffraction angles 20 of 4.83±0.2°, 5.61±0.2°, 6.85±0.2°, 8.51±0.2°, 10.16±0.2°, 14.54±0.2°, and 17.01±0.2°.

In some embodiments, the crystal form II has an X-ray powder diffraction pattern comprising characteristic peaks at diffraction angles 20 of 4.83±0.2°, 5.61±0.2°, 6.85±0.2°, 8.51±0.2°, 10.16±0.2°, 14.54±0.2°, and 17.01±0.2°.

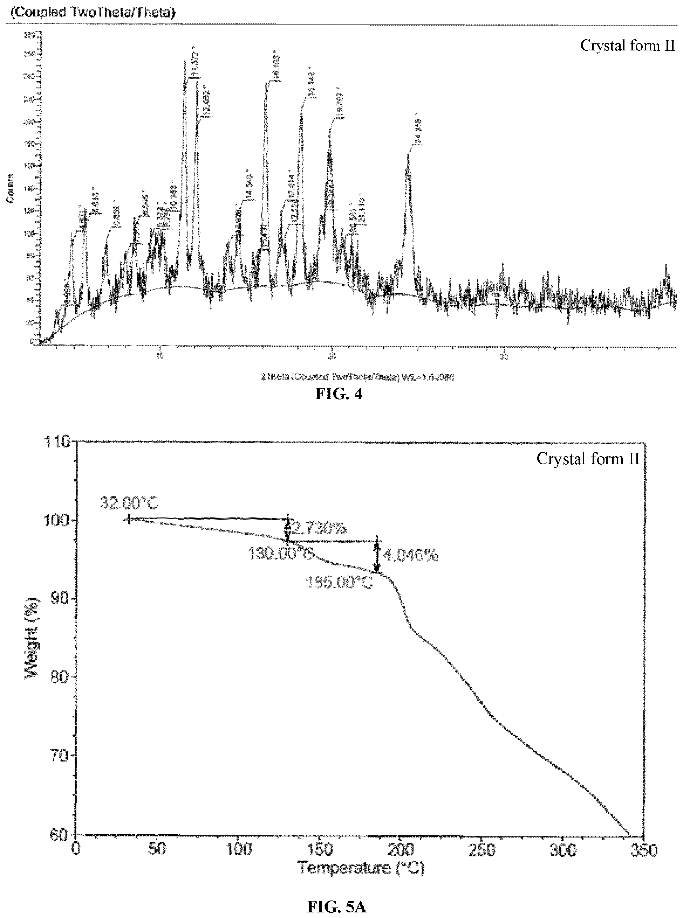

In some embodiments, the crystal form II has an X-ray powder diffraction pattern substantially consistent with FIG. 4.

In some embodiments, the method for preparing the crystal form II comprises crystallizing the salt of formula (I-I) in MTBE solvent to give the crystal form II.

In some embodiments, the crystal form II has a differential scanning calorimetry profile comprising endothermic peaks at 61.9 °C, 146.8 °C, and 184.6 °C.

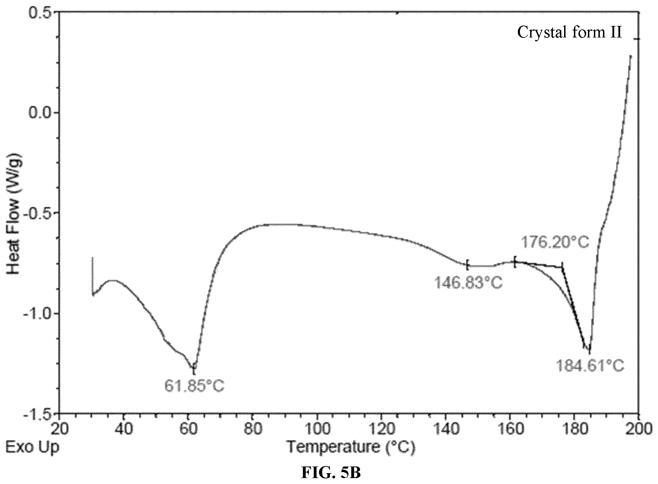

In some embodiments, the crystal form II has a differential scanning calorimetry profile substantially consistent with FIG. 5B.

In some embodiments, the triphenylacetate salt is a crystal, and the crystal is crystal form III formed by crystallizing the salt of formula (I-I): 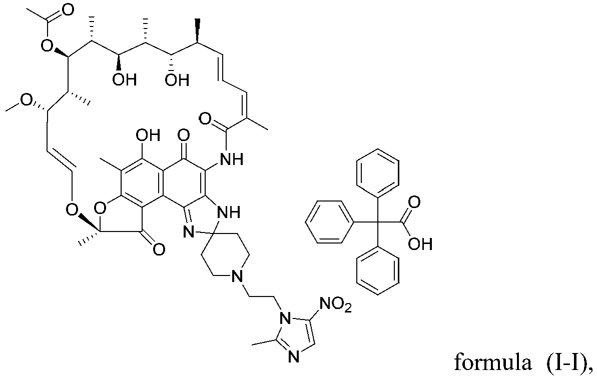 wherein the crystal III has an X-ray powder diffraction pattern comprising characteristic peaks at diffraction angles 2θ of 9.57±0.2°, 11.49±0.2°, 12.14±0.2°, 16.18±0.2°, 18.26±0.2°, 19.61±0.2°, and 19.92±0.2°.

In some embodiments, the crystal form III has an X-ray powder diffraction pattern comprising characteristic peaks at one or more diffraction angles 2θ of 4.10±0.2°, 5.00±0.2°, 5.76±0.2°, 19.15±0.2°, 20.81±0.2°, 21.60±0.2°, and 24.52±0.2°.

In some embodiments, the crystal form III has an X-ray powder diffraction pattern comprising characteristic peaks at diffraction angles 2θ of 4.10±0.2°, 5.00±0.2°, 5.76±0.2°, 19.15±0.2°, 20.81±0.2°, 21.60±0.2°, and 24.52±0.2°.

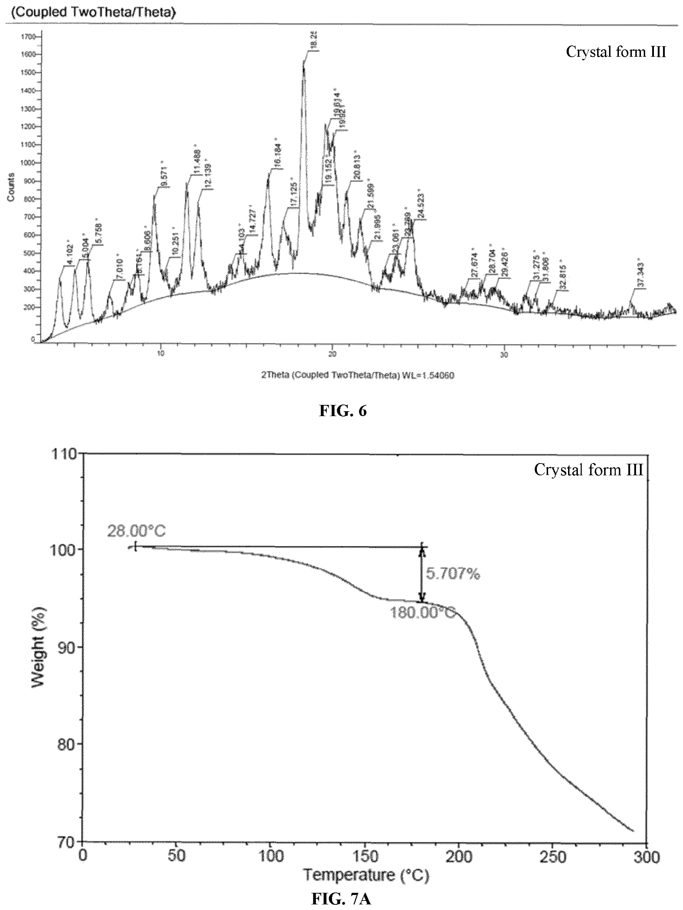

In some embodiments, the crystal form III has an X-ray powder diffraction pattern substantially consistent with FIG. 6.

In some embodiments, the method for preparing the crystal form III comprises crystallizing the salt of formula (I-I) in a solvent mixture of 2-methyltetrahydrofuran and n-heptane to give the crystal form III.

In some embodiments, the crystal form III has a differential scanning calorimetry profile comprising endothermic peaks at 64.7 °C, 142.8 °C, 159.4 °C, and 177.0 °C.

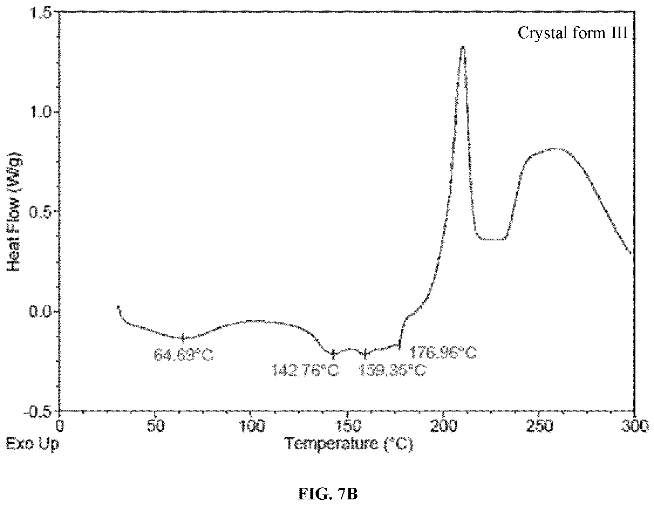

In some embodiments, the crystal form III has a differential scanning calorimetry profile substantially consistent with FIG. 7B.

In some embodiments, the triphenylacetate salt is a crystal, and the crystal is crystal form IV formed by crystallizing the salt of formula (I-I): 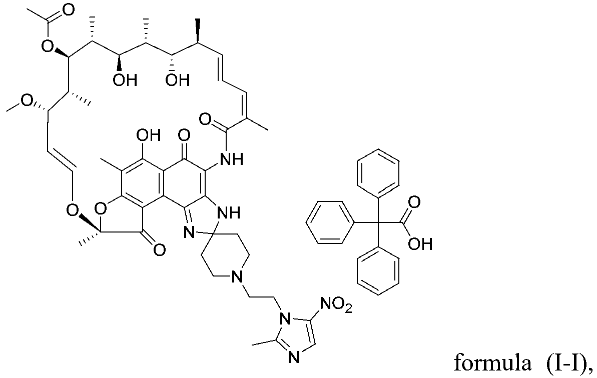 wherein the crystal IV has an X-ray powder diffraction pattern comprising characteristic peaks at diffraction angles 20 of 10.64±0.2°, 11.54±0.2°, 14.64±0.2°, 16.06±0.2°, 18.08±0.2°, and 23.89±0.2°.

In some embodiments, the crystal form IV has an X-ray powder diffraction pattern comprising characteristic peaks at one or more diffraction angles 20 of 4.85±0.2°, 7.21±0.2°, 18.82±0.2°, 20.09±0.2°, 24.26±0.2°, and 30.24±0.2°.

In some embodiments, the crystal form IV has an X-ray powder diffraction pattern comprising characteristic peaks at diffraction angles 20 of 4.85±0.2°, 7.21±0.2°, 18.82±0.2°, 20.09±0.2°, 24.26±0.2°, and 30.24±0.2°.

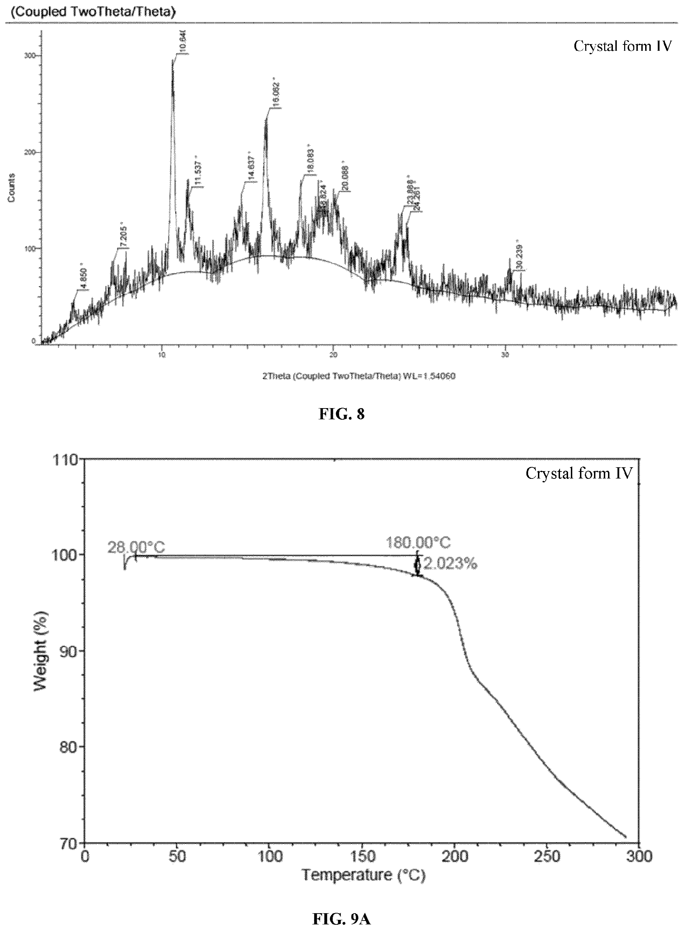

In some embodiments, the crystal form IV has an X-ray powder diffraction pattern substantially consistent with FIG. 8.

In some embodiments, the method for preparing the crystal form IV comprises heating the crystal form according to any one of claims 26-32 to 160 °C to form the crystal form IV.

In some embodiments, the crystal form IV has a differential scanning calorimetry profile comprising an endothermic peak at 182.8 °C.

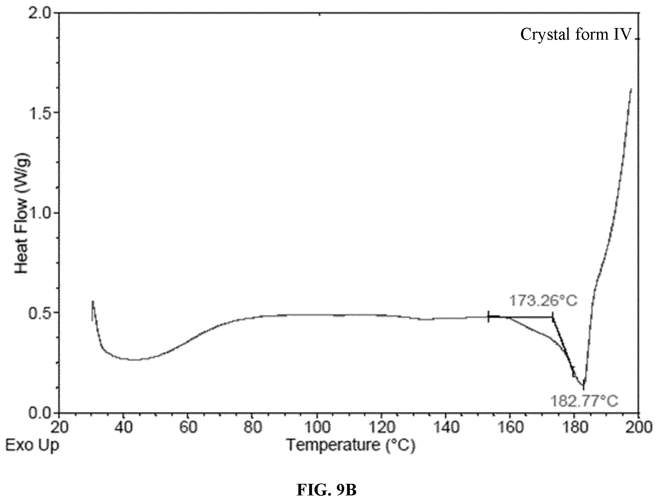

In some embodiments, the crystal form IV has a differential scanning calorimetry profile substantially consistent with FIG. 9B.

In some embodiments, the triphenylacetate salt is a crystal, and the crystal is crystal form V formed by crystallizing the salt of formula (I-II): 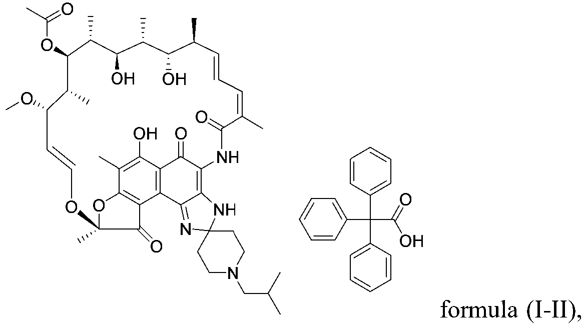 wherein the crystal form V has an X-ray powder diffraction pattern comprising characteristic peaks at diffraction angles 20 of 7.65±0.2°, 10.84±0.2°, 11.71±0.2°, 12.26±0.2°, 20.60±0.2°, and 24.83±0.2°.

In some embodiments, the crystal form V has an X-ray powder diffraction pattern comprising characteristic peaks at one or more diffraction angles 20 of 6.45±0.2°, 16.44±0.2°, 18.18±0.2°, 18.47±0.2°, and 26.54±0.2°.

In some embodiments, the crystal form V has an X-ray powder diffraction pattern comprising characteristic peaks at diffraction angles 20 of 6.45±0.2°, 16.44±0.2°, 18.18±0.2°, 18.47±0.2°, and 26.54±0.2°.

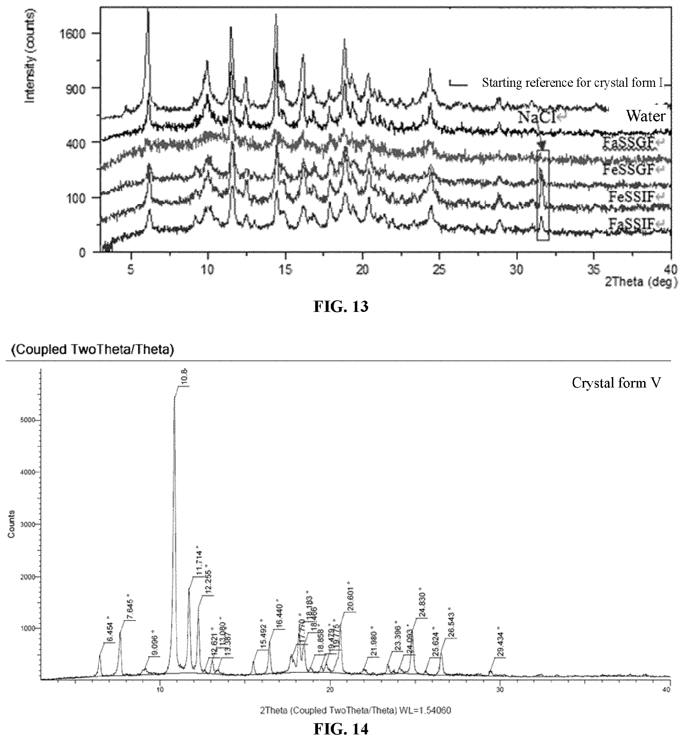

In some embodiments, the crystal form V has an X-ray powder diffraction pattern substantially consistent with FIG. 14.

In some embodiments, the method for preparing the crystal form V comprises crystallizing the triphenylacetate salt of formula (I-II) in ethyl acetate solvent to give the crystal form V.

In some embodiments, the crystal form V has a differential scanning calorimetry profile comprising an endothermic peak at 164.0 °C.

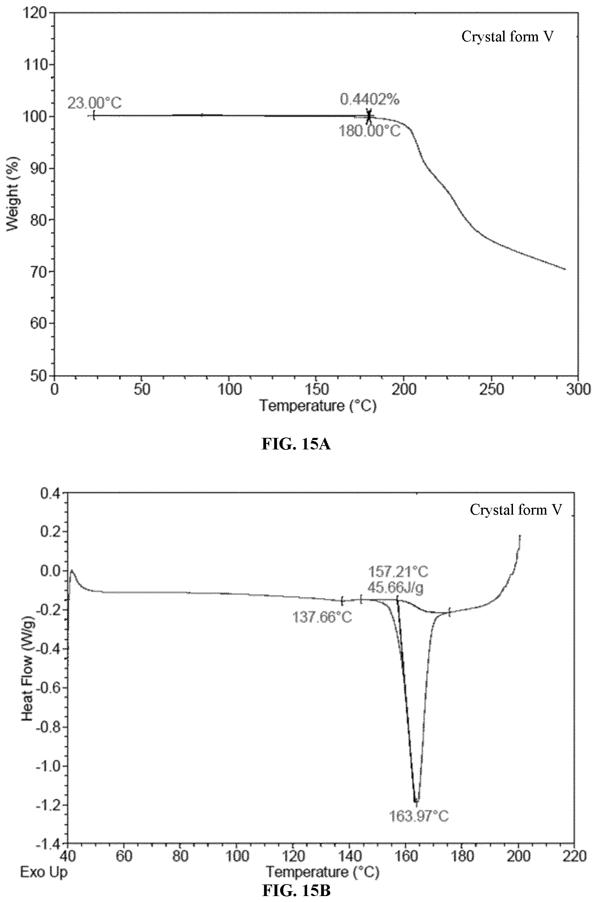

In some embodiments, the crystal form V has a differential scanning calorimetry profile substantially consistent with FIG. 15B.

In some embodiments, the triphenylacetate salt is a crystal, and the crystal is crystal form VI formed by crystallizing the salt of formula (I-II): 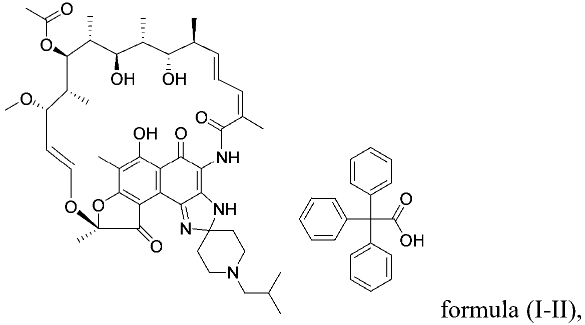 wherein the crystal form VI has an X-ray powder diffraction pattern comprising characteristic peaks at diffraction angles 2θ of 11.05±0.2°, 11.76±0.2°, 12.45±0.2°, 16.45±0.2°, 18.15±0.2°, 20.59±0.2°, and 24.77±0.2°.

In some embodiments, the crystal form VI has an X-ray powder diffraction pattern comprising characteristic peaks at one or more diffraction angles 20 of 5.89±0.2°, 6.63±0.2°, 8.87±0.2°, 9.31±0.2°, 17.87±0.2°, 20.00±0.2°, and 24.26±0.2°.

In some embodiments, the crystal form VI has an X-ray powder diffraction pattern comprising characteristic peaks at diffraction angles 20 of 5.89±0.2°, 6.63±0.2°, 8.87±0.2°, 9.31±0.2°, 17.87±0.2°, 20.00±0.2°, and 24.26±0.2°.

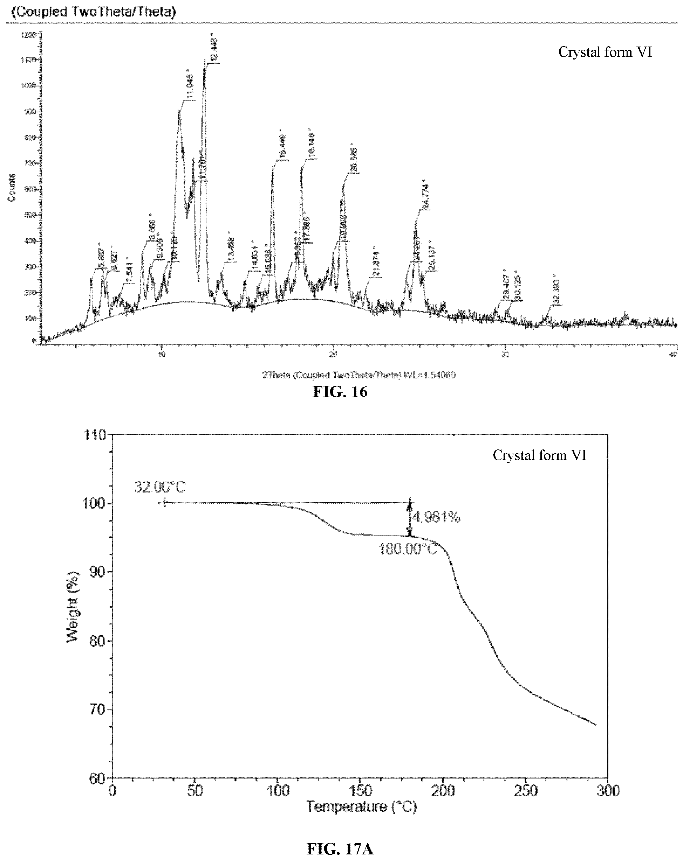

In some embodiments, the crystal form VI has an X-ray powder diffraction pattern substantially consistent with FIG. 16.

In some embodiments, the method for preparing the crystal form VI comprises crystallizing the triphenylacetate salt of formula (I-II) in methyl tert-butyl ether solvent to give the crystal form VI.

In some embodiments, the crystal form VI has a differential scanning calorimetry profile comprising endothermic peaks at 127.2 °C, 140.6 °C, and 161.8 °C.

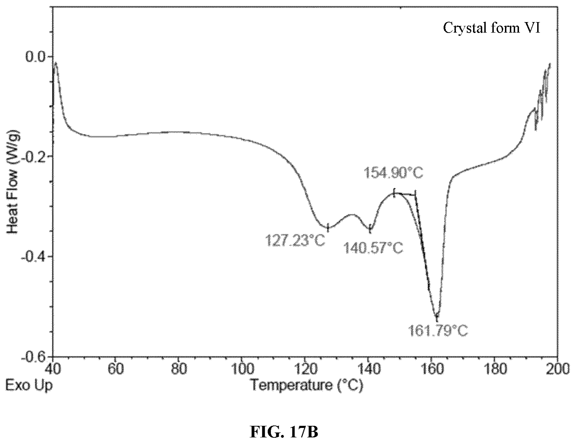

In some embodiments, the crystal form VI has a differential scanning calorimetry profile substantially consistent with FIG. 17B.

In another aspect, the present application provides a method for preparing a salt, comprising: reacting a free base of the compound of formula (I) with triphenylacetic acid to form the triphenylacetate salt of the present application.

In some embodiments, the method comprises reacting the free base of the compound of formula (I) with triphenylacetic acid in a solvent or a combination consisting of solvents selected from: (1) ethyl acetate, (2) 2-methyltetrahydrofuran and isopropyl acetate, (3) 2-methyltetrahydrofuran and n-heptane, (4) 2-butanone and n-heptane, (5) methyl tert-butyl ether, (6) methyl tert-butyl ether and n-heptane, (7) toluene, (8) isopropanol and n-heptane, (9) isopropyl acetate, (10) acetone, (11) methanol and methyl tert-butyl ether, and (12) n-heptane.

In some embodiments, the method comprises mixing the free base of the compound of formula (I), ethyl acetate, and triphenylacetic acid sequentially to give a reaction mixture.

In some embodiments, the method comprises incubating the reaction mixture at a reaction temperature of 20-40 °C.

In some embodiments, the method comprises mixing triphenylacetic acid, 2-methyltetrahydrofuran, the free base of the compound of formula (I), and isopropyl acetate sequentially to give a reaction mixture.

In some embodiments, the method comprises mixing triphenylacetic acid, 2-methyltetrahydrofuran, the free base of the compound of formula (I), and n-heptane sequentially to give a reaction mixture. In some embodiments, the method comprises incubating the reaction mixture at a reaction temperature of 50-75 °C.

In some embodiments, the method comprises mixing the free base of the compound of formula (I), 2-butanone, triphenylacetic acid, and n-heptane sequentially to give a reaction mixture.

In some embodiments, the method comprises incubating the reaction mixture at a reaction temperature of 40-60 °C.

In some embodiments, the salt formed from the free base of the compound of formula (I) and triphenylacetic acid is a crystal, and the method further comprises recrystallizing the crystal in a proper solvent to increase the purity.

In some embodiments, the proper solvent comprises 2-butanone and n-heptane, or comprises acetone and n-heptane.

In another aspect, the present application provides a method of preparing a medicament, comprising: providing the triphenylacetate salt of the present application.

In some embodiments, the method comprises: 1) dissolving the triphenylacetate salt in a proper organic solvent to convert the salt into a free base, 2) adjusting the free base to a proper concentration using a proper organic solvent, and 3) using an organic solvent capable of precipitating a purified drug solid from a solution of the free base to give the drug.

In some embodiments, the proper organic solvents in steps 1) and 2) are each independently selected from: dichloromethane, ethyl acetate, isopropyl acetate, methyl tert-butyl ether, and 2-methyltetrahydrofuran, and the organic solvents used in steps 1) and 2) have different solubilities.

In some embodiments, the solvent capable of precipitating the purified drug solid is n-heptane.

In some embodiments, the method comprises: 1) dissolving the triphenylacetate salt of the present application in a proper organic solvent, washing one or more times with a washing solution, collecting the washed organic phase, washing the washed organic phase with water, and collecting the water-washed organic phase; 2) adding an organic solvent having a solubility different from that of the organic solvent in step 1) to the collected water-washed organic phase in step 1) and concentrating at reduced pressure, and repeating the procedure until a free base concentrate of the compound of formula (I) is acquired; and 3) adding the free base concentrate in step 3) to an organic solvent capable of precipitating the purified drug solid in a nitrogen atmosphere, filtering, washing, and drying to give the drug.

In some embodiments, the washing solution is selected from a proper weak base solution.

In some embodiments, the washing solution is a sodium bicarbonate solution, a sodium carbonate solution, a potassium bicarbonate solution, or a potassium carbonate solution.

In some embodiments, the residue of the proper organic solvent in step 1) in the free base concentrate is equal to or less than 10.0%.

In some embodiments, step 3) comprises dropwise adding the free base concentrate into n-heptane with the internal temperature kept at 10-20 °C while stirring over a period of 3-8 hours, and continuously stirring for 1-2 hours after the dropwise addition.

In another aspect, the present application provides a pharmaceutical composition comprising the triphenylacetate salt of the present application and optionally a pharmaceutically acceptable carrier.

In some embodiments, the pharmaceutical composition comprises a second therapeutic agent.

In some embodiments, the second therapeutic agent is selected from: a proton pump inhibitor, an acid inhibitor, an efflux pump inhibitor, an anesthetic, an antifungal agent, an antiviral agent, an antibacterial agent, an antiprotozoal agent, an anti-inflammatory agent, an anticoagulant, a platelet aggregation inhibitor, an antipyretic, a lipid-lowering agent, and a zinc salt.

In another aspect, the present application provides a kit comprising the triphenylacetate salt of the present application and/or the pharmaceutical composition of the present application.

In some embodiments, the kit comprises written instructions and/or machine-readable electronic instructions.

In another aspect, the present application provides a method for inhibiting or preventing bacterial growth, comprising: administering an effective amount of the triphenylacetate salt of the present application, the pharmaceutical composition of the present application, and/or the kit of the present application.

In another aspect, the present application provides a method for treating a disease, comprising: administering to a patient in need an effective amount of the triphenylacetate salt of the present application, the pharmaceutical composition of the present application, and/or the kit of the present application.

In some embodiments, the patient is infected with a bacterium.

In some embodiments, the bacterium is selected from: *Helicobacter pylori, Mycobacterium tuberculosis, Nontuberculous mycobacteria, Acinetobacter baumannii, Bacteroides fragilis, Bifidobacterium longum, Ruminococcus spp., Prevotella spp., Clostridium perfringens, Clostridium difficile, Lactobacillus acidophilus, Eggertella lenta, Fusobacterium nucleatum, Gardnerella vaginalis, Mobiluncus mulieris, Peptostreptococcus spp., Porphyromonas asaccharolyticus, Prevotella bivia, Propionibacterium acnes,* and *Veillonella parvula.*

The triphenylacetate salt of the present application, the pharmaceutical composition of the present application, and/or the kit of the present application are for use in inhibiting or preventing bacterial growth.

Use of the triphenylacetate salt of the present application, the pharmaceutical composition of the present application, and/or the kit of the present application in preparing a medicament for treating and/or preventing a disease or condition caused by bacterial infection.

In some embodiments, the bacterium is selected from: *Helicobacter pylori, Mycobacterium tuberculosis, Nontuberculous mycobacteria, Acinetobacter baumannii, Bacteroides fragilis, Bifidobacterium longum, Ruminococcus spp., Prevotella spp., Clostridium perfringens, Clostridium difficile, Lactobacillus acidophilus, Eggertella lenta, Fusobacterium nucleatum, Gardnerella vaginalis, Mobiluncus mulieris, Peptostreptococcus spp., Porphyromonas asaccharolyticus, Prevotella bivia, Propionibacterium acnes,* and *Veillonella parvula.*

In some embodiments, the disease or condition is selected from: gastritis, gastric ulcer, bacterial vaginosis, diarrhea, pneumonia, appendicitis, cholecystitis, otitis media, endocarditis, endometritis, brain abscess, myocardial necrosis, osteomyelitis, peritonitis, empyema, salpingitis, septic arthritis, liver abscess, sinusitis, pelvic inflammatory disease, and bacteremia; and upper respiratory tract infection, lower respiratory tract infection, skin and soft tissue infection, bone and joint infection, lung infection, intra-abdominal infection, eye infection, ear infection, oral infection, and surgical infection.

Other aspects and advantages of the present application will be readily apparent to those skilled in the art from the following detailed description. Only exemplary embodiments of the present application have been shown and described in the following detailed description. As those skilled in the art will recognize, the content of the present application enables those skilled in the art to make changes in the specific embodiments disclosed without departing from the spirit and scope of the invention to which the present application relates. Accordingly, the drawings and description of the present application are provided only for the purpose of illustration rather than limitation.

### BRIEF DESCRIPTION OF THE DRAWINGS

The specific features of the invention to which the present application relates are set forth in the appended claims. The features and advantages of the invention to which the present application relates may be more fully understood with reference to the exemplary embodiments and drawings described in detail below. The drawings are briefly described below:
FIGs. 1A-C show the XRPD pattern, the TGA/mDSC profile, and the ¹H NMR spectrum of a starting sample of an exemplary compound of the present application, respectively.
FIG. 2 shows the XRPD pattern of the crystal form I of the present application.
FIGs. 3A and B show the TGA profile and the DSC profile of the crystal form I of the present application.
FIG. 4 shows the XRPD pattern of the crystal form II of the present application.
FIGs. 5A and B show the TGA profile and the DSC profile of the crystal form II of the present application.
FIG. 6 shows the XRPD pattern of the crystal form III of the present application.
FIGs. 7A and B show the TGA profile and the DSC profile of the crystal form III of the present application.
FIG. 8 shows the XRPD pattern of the crystal form IV of the present application.
FIGs. 9A and B show the TGA profile and the DSC profile of the crystal form IV of the present application.
FIGs. 10A-D show the ¹H NMR spectra of the crystal forms I-IV of the present application.

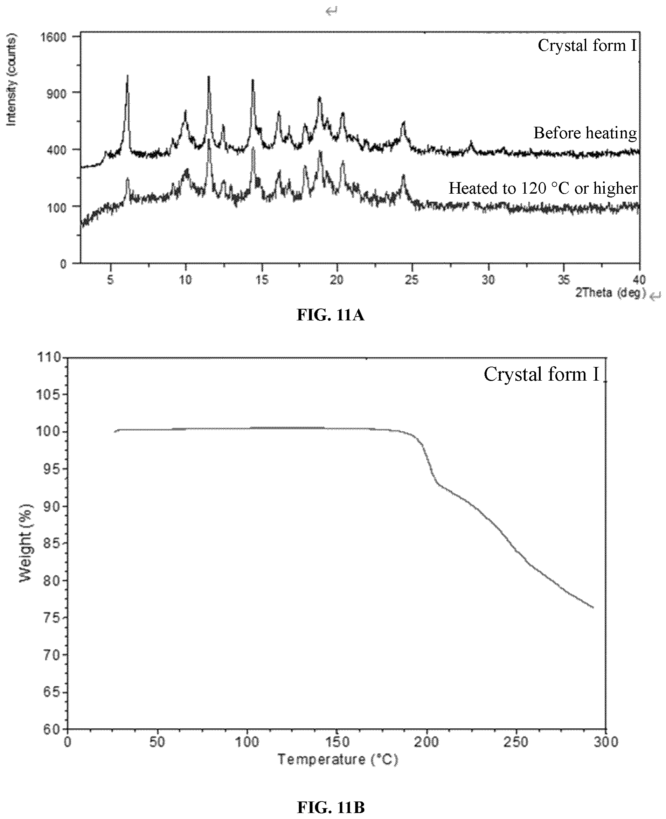

FIGs. 11A and B show the XRPD pattern and the TGA profile of the crystal form I of the present application at a high temperature (120 °C).
FIGs. 12A and B show the evaluation results of the crystal form I of the present application by DVS.
FIG. 13 shows the XRPD pattern of the crystal form I of the present application in water and four biological media (after 24 h).
FIG. 14 shows the XRPD pattern of the crystal form V of the present application.
FIGs. 15A and B show the TGA profile and the DSC profile of the crystal form V of the present application.
FIG. 16 shows the XRPD pattern of the crystal form VI of the present application.
FIGs. 17A and B show the TGA profile and the DSC profile of the crystal form VI of the present application.
FIG. 18 shows the XRPD pattern comparison of the crystal forms V and VI of the present application with the amorphous form of rifabutin.
FIG. 19 shows the morphology of the crystal form V and the crystal form VI of the present application by polarized light microscopy (PLM).

### DETAILED DESCRIPTION

The implementation of the invention of the present application is described below with reference to specific embodiments, and other advantages and effects of the invention of the present application will be readily apparent to those skilled in the art from the disclosure of the specification.

### Definitions of Terms

In the present application, the term "solvate" generally refers to a coordination complex or complex of one or more solvent molecules with the salt form of the compound of the present application. Examples of the solvents for forming solvates include, but are not limited to, water, isopropanol, ethanol, methanol, DMSO, ethyl acetate, acetic acid, and ethanolamine. The term "hydrate" generally refers to a complex in which the solvent molecule is water.

In the present application, the term "prodrug" generally refers to that the prodrug may be administered to a subject and then metabolized to form the compound of the present application; the term "metabolite" generally refers to a substance obtained by administration of the compound of the present application to a subject and metabolism. Prodrugs and metabolites encompassed in such derivatives are included within the scope of the invention of the present application.

In the present application, the terms "deuteride" and "deuterated compound" are used interchangeably and generally refer to a novel compound obtained by substituting one or more hydrogen atoms in an organic compound with deuterium atoms.

In the present application, the term "hydrogen" generally refers to a single hydrogen atom. Such a radical may be connected to other groups, such as to an oxygen atom, to form a hydroxyl group.

In the present application, the term "alkyl" generally refers to a residue derived from an alkane by the removal of a hydrogen atom. The alkyl may be substituted or unsubstituted, or replaced or unreplaced. The term "alkyl" generally refers to a saturated linear or branched aliphatic hydrocarbon group having a residue derived from the parent alkane by removal of hydrogen atoms from the same carbon atom or two different carbon atoms, and it may be a linear or branched group containing 1 to 20 carbon atoms, e.g., 1 to 12 carbon atoms, such as a chain alkyl containing 1 to 6 carbon atoms. Non-limiting examples of the alkyl include, but are not limited to, methyl, ethyl, propyl, butyl, etc. The alkyl may be substituted or unsubstituted, or replaced or unreplaced. For example, when substituted, the alkyl may be substituted at any available connection site with a substituent that may be independently, optionally selected from one or more of alkyl, alkenyl, alkynyl, alkoxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo.

In the present application, the term "aryl" generally refers to a group having a residue derived from an aromatic ring by the removal of a hydrogen atom. The term "aromatic ring" may refer to a 6- to 14-membered all-carbon monocyclic ring or fused polycyclic ring (i.e., rings that share a pair of adjacent carbon atoms) having a conjugated π-electron system, which may be 6- to 10-membered, such as benzene and naphthalene. The aromatic ring can be fused to a heteroaryl, heterocyclyl, or cycloalkyl ring, wherein the ring connected to the parent structure is the aryl ring. The aryl may be substituted or unsubstituted, and when it is substituted, the substituent may be one or more of the following groups independently selected from: alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio. The aryl groups may be optionally substituted as described herein.

In the present application, the term "alkenyl" generally refers to a linear or branched hydrocarbon group containing one or more double bonds. Exemplary instances of alkenyl include allyl, homoallyl, vinyl, crotyl, butenyl, pentenyl, hexenyl, etc. Exemplary instances of C₂₋₆ chain alkenyl containing one or more double bonds include butadienyl, pentadienyl, hexadienyl, and hexatrienyl, as well as branched forms thereof. The position of the unsaturated bond (double bond) may be any position in the carbon chain. The alkenyl may be substituted or unsubstituted.

In the present application, the term "alkynyl" generally refers to unsaturated linear or branched alkynyl, e.g., ethynyl, 1-propynyl, propargyl, butynyl, etc. The alkynyl may be substituted or unsubstituted.

In the present application, the term "optionally substituted" generally refers to that the referenced group may be substituted or unsubstituted with one or more additional groups individually and independently selected from alkyl, alkenyl, alkynyl, cycloalkyl, aryl, heteroaryl, heterocycloalkyl, hydroxy, alkoxy, thiol, cyano, halogen, carbonyl, thiocarbonyl, isocyanato, thiocyanate, isothiocyanato, nitro, perhaloalkyl, perfluoroalkyl, and amino including mono-substituted and disubstituted amino groups, and the protected derivatives thereof. The position and number of such substituent groups are determined by the well-known valence limitations of the groups.

In the present application, the term "cycloalkyl" generally refers to "alkyl" in a cyclic form including saturated monocyclic, bicyclic, or polycyclic alkyl groups. The rings of the bicyclic or polycyclic ring systems may be fused and linked through a single shared atom, i.e., they form a spiro or bridged ring system. The cycloalkyl may contain 3 to 10 carbon atoms, e.g., 3, 4, 5, 6, 7, 8, 9 or 10 carbon atoms to form the ring. Examples of suitable cycloalkyl include, but are not limited to, cyclopropyl, cyclobutyl, cyclopentyl, cyclohexyl, cycloheptyl, cyclooctyl, cyclononyl, or cyclodecyl. Examples of suitable cycloalkyl include, but are not limited to, cyclopropyl, cyclobutyl, cyclopentyl, cyclohexyl, cycloheptyl, cyclooctyl, spiro[3,3]heptyl, spiro[3,4]octyl, spiro[4,3]octyl, bicyclo[4.1.0]heptyl, bicyclo[3.2.0]heptyl, bicyclo[2.2.1]heptyl, bicyclo[2.2.2]octyl, bicyclo[5.1.0]octyl, or bicyclo[4.2.0]octyl. The cycloalkyl may be optionally substituted as described herein.

In the present application, the term "heterocyclyl" generally refers to a monocyclic, bicyclic or tricyclic, saturated or partially unsaturated, non-aromatic ring system having 3 to 20 ring atoms, including fused ring systems, in which at least one ring atom is a heteroatom. Examples of the heteroatom include nitrogen, oxygen, and sulfur. In some embodiments, the heterocyclyl refers to a saturated ring system, such as a 3- to 12-membered saturated heterocyclyl ring system, or a 3- to 8-membered saturated heterocyclyl ring system. In some embodiments, the heterocyclyl refers to a 5-to 8-membered saturated heterocyclyl ring system. In some embodiments, the heterocyclyl refers to a 5- to 6-membered saturated heterocyclyl ring system. In some embodiments, the heterocyclyl comprises 1 to 4 heteroatoms. In some embodiments, the heterocyclyl comprises a 3- to 7-membered monocyclic ring having one or more heteroatoms selected from nitrogen, oxygen, and sulfur. In some embodiments, the heterocyclyl comprises a 4- to 6-membered monocyclic ring having one or more heteroatoms selected from nitrogen, oxygen, and sulfur. In another example, the heterocyclyl comprises a 3-membered monocyclic ring. In another example, the heterocyclyl comprises a 4-membered monocyclic ring. In another example, the heterocyclyl comprises a 5- to 6-membered monocyclic ring. In one example, the heterocyclyl comprises 0 to 3 double bonds.

In the present application, the term "heteroatom" generally refers to nitrogen, oxygen, or sulfur, and includes any oxidized form of nitrogen or sulfur and any quaternized form of basic nitrogen.

In the present application, as known to those skilled in the art, the terms such as "alkyl", "alkenyl", and "cycloalkyl" may be preceded by a notation to indicate the number of atoms present in the group under particular circumstances as in C₁-C₄ alkyl, C₃-C₇ cycloalkoxy, C₁-C₄ alkylcarbonylamino, etc., and the subscript numeral following "C" indicates the number of carbon atoms present in the group. For example, the C₃ alkyl refers to an alkyl group having three carbon atoms (e.g., n-propyl or isopropyl); in C₁₋₁₀, members of the group may have any number of carbon atoms within the range of 1-10.

In the present application, the term "pharmaceutically acceptable carrier" generally refers to a pharmaceutically acceptable substance, composition, or vehicle involved in carrying or transporting a chemical agent, such as a liquid or solid filler, diluent, excipient, solvent, or encapsulating material. The pharmaceutically acceptable carrier includes pharmaceutically acceptable salts, wherein the term "pharmaceutically acceptable salt" includes salts of the active compound prepared using relatively non-toxic acids or bases, depending on the particular substituents found on the compound described herein. When the compound of the invention of the present application contains relatively acidic functional groups, a base addition salt can be obtained by contacting the neutral form of such compounds with a sufficient amount of a desired base in a net manner or in a suitable inert solvent. Examples of the pharmaceutically acceptable base addition salt include sodium, potassium, calcium, ammonium, organic amino or magnesium salts, or similar salts. When the compound of the invention of the present application contains relatively basic functional groups, an acid addition salt can be obtained by contacting the neutral form of such compounds with a sufficient amount of a desired acid in a net manner or in a suitable inert solvent. Examples of the pharmaceutically acceptable acid addition salt include salts derived from inorganic acids and from relatively non-toxic organic acids. Certain specific compounds of the invention of the present application contain basic and acidic functional groups capable of converting the compound into a base or acid addition salt.

In the present application, the term "bacterium" generally refers to a class of prokaryotic organisms without distinct nuclei and membranous organelles. The bacterium may include spherical, rod-shaped, or spiral species. The bacteria may include species that grow interlike, for example, *Escherichia, Salmonella, Shigella, Klebsiella, Vibrio, Pasteurella, Borrelia, Leptospira, Campylobacter, Clostridium, Corynebacterium, Yersinia, Treponema, Rickettsia, Chlamydia, Mycoplasma, Coxiella, Neisseria, Listeria, Haemophilus, Helicobacter, Legionella, Pseudomonas, Bordetella, Brucella, Staphylococcus, Streptococcus, Enterococcus, Bacillus, Mycobacterium, Nocardia,* etc. The term "bacterial infection" generally refers to any disorder caused by the expansion and/or presence of a bacterium described herein in a cell or a subject. The bacterial infection can be caused by the growth and reproduction of a bacterium (e.g., a pathogenic bacterium) that produces toxins and other metabolic products.

In the present application, the term "subject in need" generally refers to any organism to which the compound and/or composition described herein can be administered, e.g., for experimental, diagnostic, prophylactic, and/or therapeutic purposes. Typical subjects include animals (e.g., mammals, such as mice, rats, rabbits, dogs, non-human primates, and humans) and/or plants.

In the present application, the term "effective amount" generally refers to an amount sufficient to achieve, or at least partially achieve, the desired therapeutic effect. The "effective amount" of a drug or therapeutic agent generally refers to an amount sufficient to cure, or at least partially arrest, the disease and its complications in a patient already suffering from the disease. The effective amount for this use will depend on the severity of the infection and the overall state of the patient's own immune system.

In the present application, the term "comprise" or "comprising" generally refers to the inclusion of the explicitly specified features without excluding other elements. The terms "not less than" and "not more than" generally refer to the circumstance where the number itself is included.

In the present application, the term "selected from" generally refers to the inclusion of the selected objects and all combinations thereof. For example, "selected from A, B, and C" refers to the inclusion of all combinations of A, B, and C, e.g., A, B, C, A+B, A+C, B+C, or A+B+C.

### Detailed Description of the Invention

### Compound and Salt Form Thereof

In one aspect, the present application provides triphenylacetate salts of the compounds of formula (I), or hydrates, solvates, prodrugs, metabolites or deuterated compounds thereof: 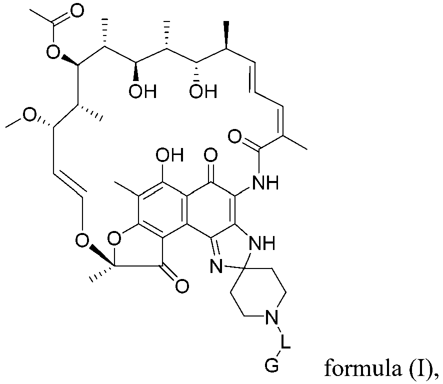 wherein L is a bond, alkyl, or the structure shown in 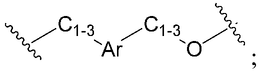 Ar is optionally substituted aryl; G is hydrogen, alkyl, haloalkyl, alkenyl, alkynyl, optionally substituted cycloalkyl, or optionally substituted heterocyclyl.

In some embodiments, the molar ratio of the compound of formula (I) to triphenylacetic acid is 0.8-1.2. For example, the molar ratio of the compound of formula (I) to triphenylacetic acid may be 0.80, 0.90, 1.00, 1.10, 1.11, 1.12, 1.13, 1.14, 1.15, 1.16, 1.17, 1.18, 1.19, or 1.20. More specifically, the compound of formula (I) and triphenylacetic acid are in a molar ratio of 1:1, and the formed salt is a monotriphenylacetate salt.

In some embodiments, L may be C₁-C₆ alkyl. For example, L may be C₁ alkyl, C₂ alkyl, C₃ alkyl, C₄ alkyl, C₅ alkyl, or C₆ alkyl. For example, the structure of L may also be:

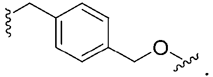

In some embodiments, G may be C₁-C₆ alkyl, C₂-C₆ alkenyl, or C₂-C₆ alkynyl. For example, G may be C₁ alkyl, C₂ alkyl, C₃ alkyl, C₄ alkyl, C₅ alkyl, C₆ alkyl, C₂ alkenyl, C₃ alkenyl, C₄ alkenyl, C₅ alkenyl, C₆ alkenyl, C₂ alkynyl, C₃ alkynyl, C₄ alkynyl, C₅ alkynyl, or C₆ alkynyl.

In some embodiments, G may be C₁-C₃ monohaloalkyl or C₁-C₃ dihaloalkyl. For example, G may be C₁ monohaloalkyl, C₂ monohaloalkyl, C₃ monohaloalkyl, C₁ dihaloalkyl, C₂ dihaloalkyl, or C₃ dihaloalkyl.

In some embodiments, G may also be optionally substituted C₃-C₈ cycloalkyl, optionally substituted 3- to 8-membered heteroatom-containing monocyclic substituent, or optionally substituted 4- to 12-membered heteroatom-containing bicyclic substituent. For example, G may also be C₃ cycloalkyl, C₄ cycloalkyl, C₅ cycloalkyl, C₆ cycloalkyl, C₇ cycloalkyl, C₈ cycloalkyl, 3-membered heteroatom-containing monocyclic substituent, 4-membered heteroatom-containing monocyclic substituent, 5-membered heteroatom-containing monocyclic substituent, 6-membered heteroatom-containing monocyclic substituent, 7-membered heteroatom-containing monocyclic substituent, 8-membered heteroatom-containing monocyclic substituent, 4-membered heteroatom-containing bicyclic substituent, 5-membered heteroatom-containing bicyclic substituent, 6-membered heteroatom-containing bicyclic substituent, 7-membered heteroatom-containing bicyclic substituent, 8-membered heteroatom-containing bicyclic substituent, 9-membered heteroatom-containing bicyclic substituent, 10-membered heteroatom-containing bicyclic substituent, 11-membered heteroatom-containing bicyclic substituent, or 12-membered heteroatom-containing bicyclic substituent, wherein (1) all of the above G groups may be unsubstituted or substituted with any substituent, and (2) the heteroatom-containing monocyclic or bicyclic substituents may each contain 1, 2, 3, or 4 heteroatoms selected from N, O, and S. For example, for the G group that may be substituted with any substituent, the substituent may be selected from: halogen, hydroxyl, carboxyl, carbonyl, amino, nitro, thiol, cyano, alkyl, alkenyl, alkynyl, cycloalkyl, aryl, heteroaryl, alkylamino, alkoxy, and carbonyl ester group.

Illustratively, G may be any one of the following structures:

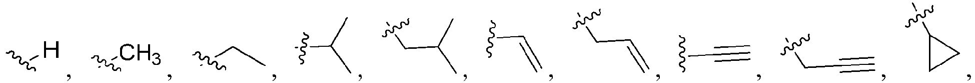

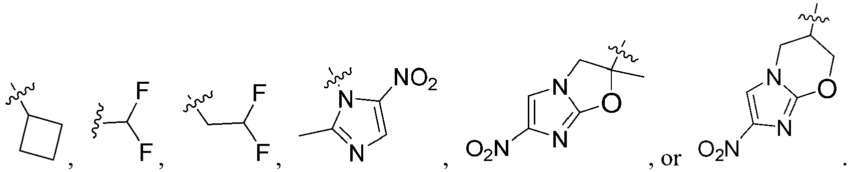

In some embodiments, an exemplary structure of 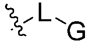 of formula (I) is any one of the following structures:

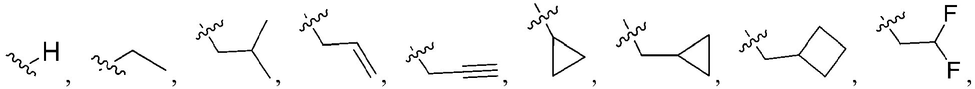

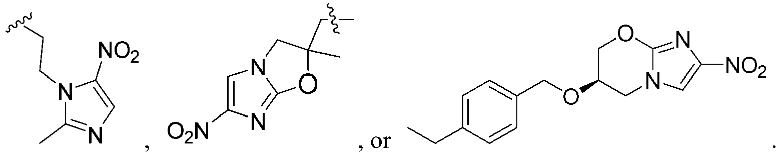

Illustratively, the compound of formula (I) may be any one of the following structures: 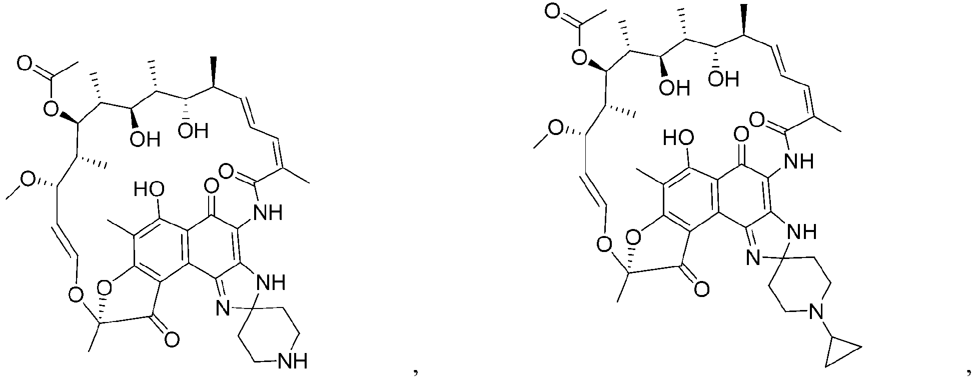 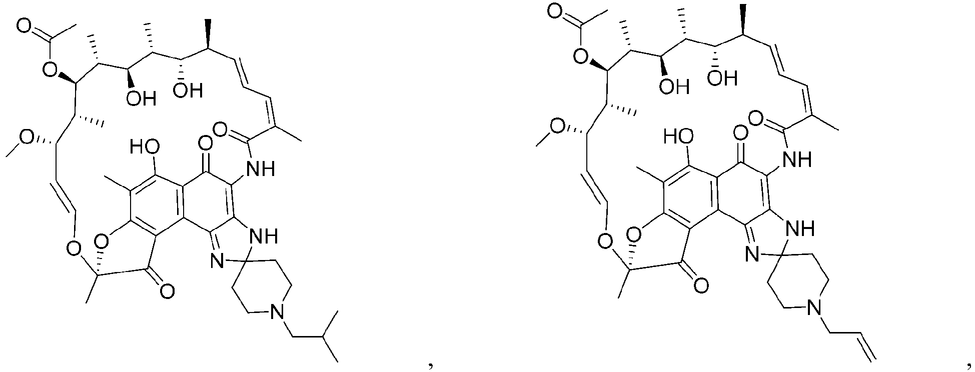 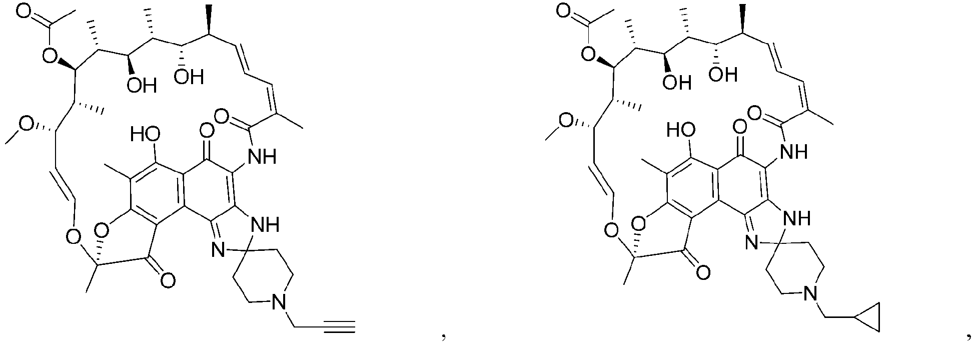 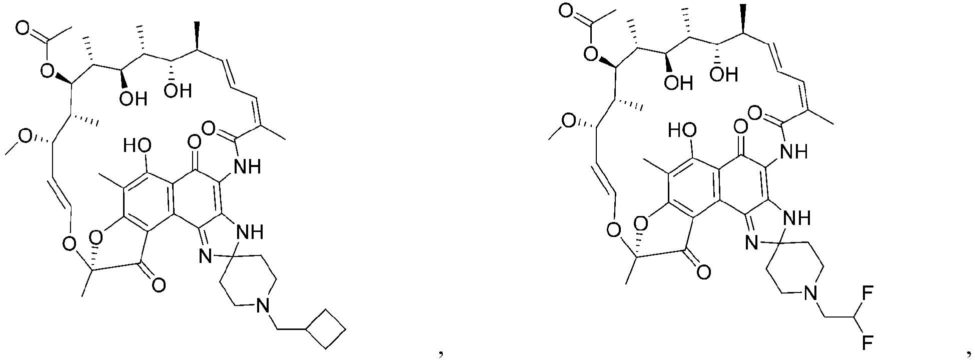 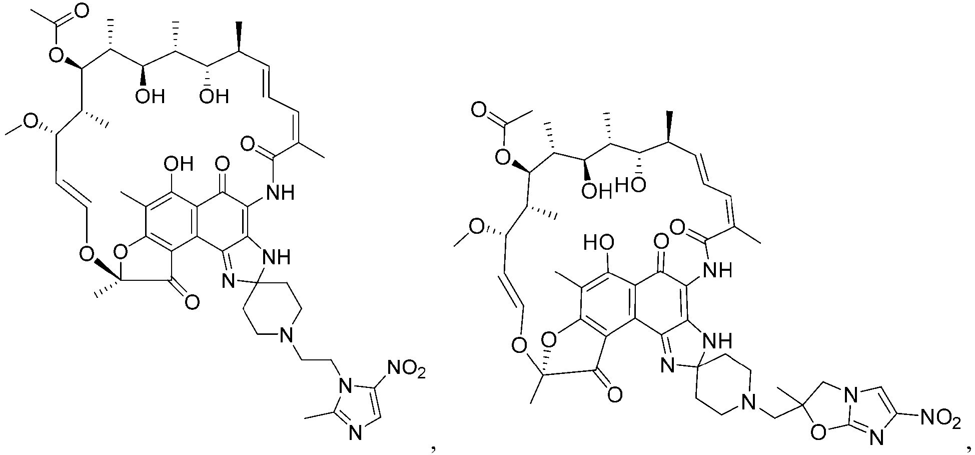 or

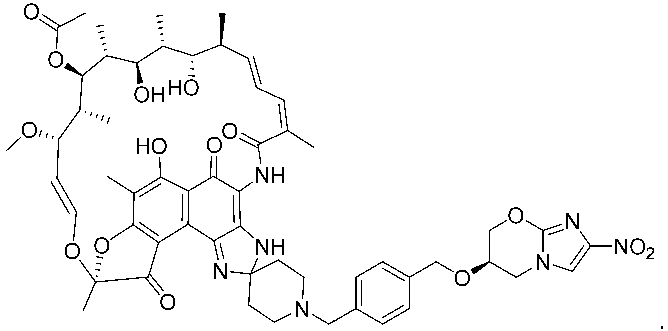

In some embodiments, the triphenylacetate salt is in a crystal, and the crystal can form crystal forms I, II, III, and IV by crystallizing a salt of formula (I-I); the crystal can also form crystal forms V and VI by crystallizing a salt of formula (I-II). The present application will help to further understand the characteristics of the crystal form and the method for preparing same through the following examples, which are not intended to limit the content of the present application.

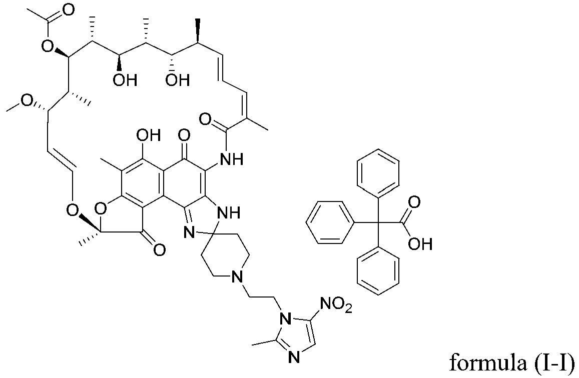

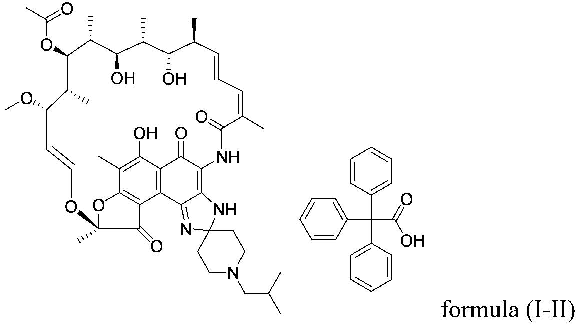

### Preparation Method

In another aspect, the present application provides a method for preparing the salts, comprising: reacting a free base of the compounds of formula (I) with triphenylacetic acid to form the salts of the present application.

In some embodiments, the method comprises reacting the free base of the compound of formula (I) with triphenylacetic acid in a solvent or a combination consisting of solvents selected from: (1) ethyl acetate, (2) 2-methyltetrahydrofuran and isopropyl acetate, (3) 2-methyltetrahydrofuran and n-heptane, (4) 2-butanone and n-heptane, (5) methyl tert-butyl ether, (6) methyl tert-butyl ether and n-heptane, (7) toluene, (8) isopropanol and n-heptane, (9) isopropyl acetate, (10) acetone, (11) methanol and methyl tert-butyl ether, and (12) n-heptane.

For example, the method may comprise mixing the free base of the compound of formula (I), ethyl acetate, and triphenylacetic acid sequentially to give a reaction mixture. Furthermore, the method comprises incubating the reaction mixture at a reaction temperature of 20-40 °C.

For example, the method may comprise mixing triphenylacetic acid, 2-methyltetrahydrofuran, the free base of the compound of formula (I), and isopropyl acetate sequentially to give a reaction mixture. Furthermore, the method comprises incubating the reaction mixture at a reaction temperature of 50-75 °C.

For example, the method may comprise mixing triphenylacetic acid, 2-methyltetrahydrofuran, the free base of the compound of formula (I), and n-heptane sequentially to give a reaction mixture. Furthermore, the method comprises incubating the reaction mixture at a reaction temperature of 50-75 °C.

For example, the method may further comprise mixing the free base of the compound of formula (I), 2-butanone, triphenylacetic acid, and n-heptane sequentially to give a reaction mixture. Furthermore, the method comprises incubating the reaction mixture at a reaction temperature of 40-60 °C.

In some embodiments, the salt formed from the free base of the compound of formula (I) and triphenylacetic acid is a crystal, and the method further comprises recrystallizing the crystal in a proper solvent to increase the purity.

In some embodiments, the proper solvent comprises 2-butanone and n-heptane, or comprises acetone and n-heptane. For example, the crystal may be subjected to one or more recrystallizations in a solvent system comprising 2-butanone and n-heptane to give a crystal form with a higher purity than the previous time; or, the crystal may also be subjected to one or more recrystallizations in a solvent system comprising acetone and n-heptane to give a crystal form with a higher purity than the previous time.

In another aspect, the present application provides a method for preparing a medicament using the triphenylacetate salt described herein.

In some embodiments, the method comprises: 1) dissolving the triphenylacetate salt in a proper organic solvent to convert the salt into a free base, 2) adjusting the free base to a proper concentration using a proper organic solvent, and 3) using an organic solvent capable of precipitating a purified drug solid from a solution of the free base to give the drug. For example, a suitable organic solvent for dissolving the salt may be selected from: ethyl acetate, isopropyl acetate, methyl tert-butyl ether, and 2-methyltetrahydrofuran. For example, the organic solvent that can adjust the free base to a suitable concentration should be one that has a higher solubility than that of the organic solvent in which the salt was initially dissolved. For example, the solvent capable of precipitating the purified drug solid may be n-heptane.

An exemplary method for preparing a medicament using the salt described herein may comprise:
1) dissolving the triphenylacetate salt of the present application in a proper organic solvent, washing one or more times with a washing solution, collecting the washed organic phase, washing the washed organic phase with water, and collecting the water-washed organic phase. For example, the washing solution may be selected from a proper weak base solution. Specifically, the washing solution may be a sodium bicarbonate solution, a sodium carbonate solution, a potassium bicarbonate solution, or a potassium carbonate solution.
2) adding an organic solvent having a solubility different from that of the organic solvent in step 1) to the collected water-washed organic phase in step 1) and concentrating at reduced pressure, and repeating the procedure until a free base concentrate of the compound of formula (I) is acquired. For example, the residue of the organic solvent in step 1) in the free base concentrate is equal to or less than 10.0%.
3) adding the free base concentrate to a solvent capable of precipitating the purified drug solid in a nitrogen atmosphere, filtering, washing, and drying to give the desired drug. Illustratively, this step may comprise dropwise adding the free base concentrate into n-heptane with the internal temperature kept at 10-20 °C while stirring over a period of 3-8 hours, and continuously stirring for 1-2 hours after the dropwise addition.

### Composition

In another aspect, the present application provides a pharmaceutical composition comprising the triphenylacetate salt of the present application and optionally a pharmaceutically acceptable carrier. For example, the composition may be in the form of a solution, an aerosol, a gel, an ointment, a spray, or a suspension, which can be prepared using known and conventional methods in the art. For example, the pharmaceutically acceptable carrier may be selected from an excipient for increasing the solubility, an auxiliary agent for enhancing the viscosity, and an auxiliary agent for enhancing the osmotic capacity.

In some embodiments, the composition may further comprise a second therapeutic agent. For example, the second therapeutic agent may be selected from an additional rifamycin or an analog thereof, a proton pump inhibitor, an acid inhibitor, an efflux pump inhibitor, an anesthetic, an antifungal agent, an antiviral agent, an antibacterial agent, an antiprotozoal agent, an anti-inflammatory agent (e.g., a non-steroidal anti-inflammatory agent or a steroid), an anticoagulant, a platelet aggregation inhibitor, an antipyretic, a lipid-lowering agent, and a zinc salt. All therapeutic agents used in the compositions of the present application can be used in the dose range currently known and used for these formulations.

### Kit

In another aspect, the present application provides a kit comprising the triphenylacetate salt of the present application and/or the pharmaceutical composition of the present application. For example, the kit may further comprise at least one container into which the salt and/or the pharmaceutical composition are placed. For example, the kit may further comprise one or more components, and may further comprise a second, third, and/or additional containers other than the container, in which the one or more components may be separately placed. For example, the kit may further comprise various combinations of the salt and/or the pharmaceutical composition in the container(s). For example, the kit may further comprise a buffering reagent, a device for mixing different components, a device for measurement, a device for sorting components, and/or a device for labeling. For example, the kit may further comprise a package for receiving the various containers. For example, the kit may further comprise instructions for using the components of the kit. For example, the instructions may include a written form and/or a machine-readable electronic form.

### Method

In another aspect, the present application provides a method for inhibiting or preventing bacterial growth, comprising: administering an effective amount of the triphenylacetate salt of the present application, the pharmaceutical composition of the present application, and/or the kit of the present application. In some embodiments, the method comprises contacting the triphenylacetate salt of the present application, the pharmaceutical composition of the present application, and/or the kit of the present application with a site infected by a bacterium. For example, the infected site may be an inner site or a surface site of an animal body, or an inner site or a surface site of a plant body.

In another aspect, the present application provides a method for treating a disease, comprising: administering to a subject in need an effective amount of the triphenylacetate salt of the present application, the pharmaceutical composition of the present application, and/or the kit of the present application. In some certain embodiments, the method may further comprise mixing the triphenylacetate salt of the present application, the pharmaceutical composition of the present application, and/or the kit of the present application with a therapeutic agent. In some embodiments, the method may comprise systemically delivering the triphenylacetate salt of the present application, the pharmaceutical composition of the present application, and/or the kit of the present application in a subject in need, so as to expose the majority of the body to the treatment. The procedure may be performed by any means known in the art, including but not limited to intravenous, intra-arterial, subcutaneous, and intraperitoneal delivery. In some other embodiments, the method may comprise locally delivering the triphenylacetate salt of the present application, the pharmaceutical composition of the present application, and/or the kit of the present application in a subject in need, so as to directly deliver the treatment to a target site in an organism or to a site infected by a bacterium on the surface of an organism, or directly contact the treatment with a site infected by a bacterium on the surface of an organism. For example, such local delivery does not exclude a systemic pharmacological effect. In some certain embodiments, the method may comprise administering to a subject in need an effective amount of the triphenylacetate salt of the present application, the pharmaceutical composition of the present application, and/or the kit of the present application by intramuscular injection, subcutaneous or intradermal injection, intravenous injection, intrathecal injection, inhalation, oral administration, topical application, etc.

In the method described above, the bacterium may be selected from: *Helicobacter pylori, Mycobacterium tuberculosis, Nontuberculous mycobacteria, Acinetobacter baumannii, Bacteroides fragilis, Bifidobacterium longum, Ruminococcus spp., Prevotella spp., Clostridium perfringens, Clostridium difficile, Lactobacillus acidophilus, Eggertella lenta, Fusobacterium nucleatum, Gardnerella vaginalis, Mobiluncus mulieris, Peptostreptococcus spp., Porphyromonas asaccharolyticus, Prevotella bivia, Propionibacterium acnes, and Veillonella parvula.* In the present application, the bacterium may further comprise a drug-resistant strain of the bacterium described above. For example, the drug-resistant strain may be resistant to rifamycins, resistant to nitroimidazoles, or resistant to both rifamycins and nitroimidazoles. For example, the drug-resistant strain may also be resistant to other antibiotics, including macrolides such as clarithromycin, azithromycin, and roxithromycin; fluoroquinolones such as ciprofloxacin, levofloxacin, and moxifloxacin; aminoglycosides such as streptomycin and amikacin; β-lactams such as ampicillin and amoxicillin; tetracyclines such as tetracycline, tigecycline, and minocycline; oxazolidinones such as linezolid and tedizolid; nitrofurans such as furazolidone; glycopeptides such as vancomycin; and diarylquinolines such as bedaquiline and clofazimine.

In addition, in some embodiments, the subject may be infected by a bacterium, or the subject may have a disease or disorder caused by a bacterial infection. For example, the disease may be selected from gastritis, gastric ulcer, bacterial vaginosis, diarrhea, pneumonia, appendicitis, cholecystitis, otitis media, endocarditis, endometritis, brain abscess, myocardial necrosis, osteomyelitis, peritonitis, empyema, salpingitis, septic arthritis, liver abscess, sinusitis, pelvic inflammatory disease, and bacteremia. For example, the disorder may be selected from upper respiratory tract infection, lower respiratory tract infection, skin and soft tissue infection, bone and joint infection, lung infection, intra-abdominal infection, eye infection, ear infection, oral infection, and surgical infection.

### Use

In another aspect, the present application provides use of the triphenylacetate salt of the present application, the pharmaceutical composition of the present application, and/or the kit of the present application in inhibiting or preventing bacterial growth.

In another aspect, the present application provides use of the triphenylacetate salt of the present application, the pharmaceutical composition of the present application, and/or the kit of the present application in preparing a medicament for treating and/or preventing a disease or condition caused by bacterial infection.

In the use described above, the bacterium may be selected from: *Helicobacter pylori, Mycobacterium tuberculosis, Nontuberculous mycobacteria, Acinetobacter baumannii, Bacteroides fragilis, Bifidobacterium longum, Ruminococcus spp., Prevotella spp., Clostridium perfringens, Clostridium difficile, Lactobacillus acidophilus, Eggertella lenta, Fusobacterium nucleatum, Gardnerella vaginalis, Mobiluncus mulieris, Peptostreptococcus spp., Porphyromonas asaccharolyticus, Prevotella bivia, Propionibacterium acnes, and Veillonella parvula.* In the present application, the bacterium may further comprise a drug-resistant strain of the bacterium described above. For example, the drug-resistant strain may be resistant to rifamycins, resistant to metronidazoles, or resistant to both rifamycins and metronidazole. For example, the drug-resistant strain may also be resistant to other antibiotics, including macrolides such as clarithromycin, azithromycin, and roxithromycin; fluoroquinolones such as ciprofloxacin, levofloxacin, and moxifloxacin; aminoglycosides such as streptomycin and amikacin; β-lactams such as ampicillin and amoxicillin; tetracyclines such as tetracycline, tigecycline, and minocycline; oxazolidinones such as linezolid and tedizolamide; nitrofurans such as furazolidone; glycopeptides such as vancomycin; and diarylquinolines such as bedaquiline and clofazimine.

In the use described above, the disease may be selected from gastritis, gastric ulcer, bacterial vaginosis, diarrhea, pneumonia, appendicitis, cholecystitis, otitis media, endocarditis, endometritis, brain abscess, myocardial necrosis, osteomyelitis, peritonitis, empyema, salpingitis, septic arthritis, liver abscess, sinusitis, pelvic inflammatory disease, and bacteremia. For example, the disorder may be selected from upper respiratory tract infection, lower respiratory tract infection, skin and soft tissue infection, bone and joint infection, lung infection, intra-abdominal infection, eye infection, ear infection, oral infection, and surgical infection.

Without being bound by any theory, the following examples are intended only to illustrate the triphenylacetate salt, the method for preparing same, the use, etc., of the present application, rather than limit the scope of the present application.

### Examples

### Example 1

### Preparation of monotriphenylacetate salt of exemplary compound

### (1) Characterization of starting samples of exemplary compound

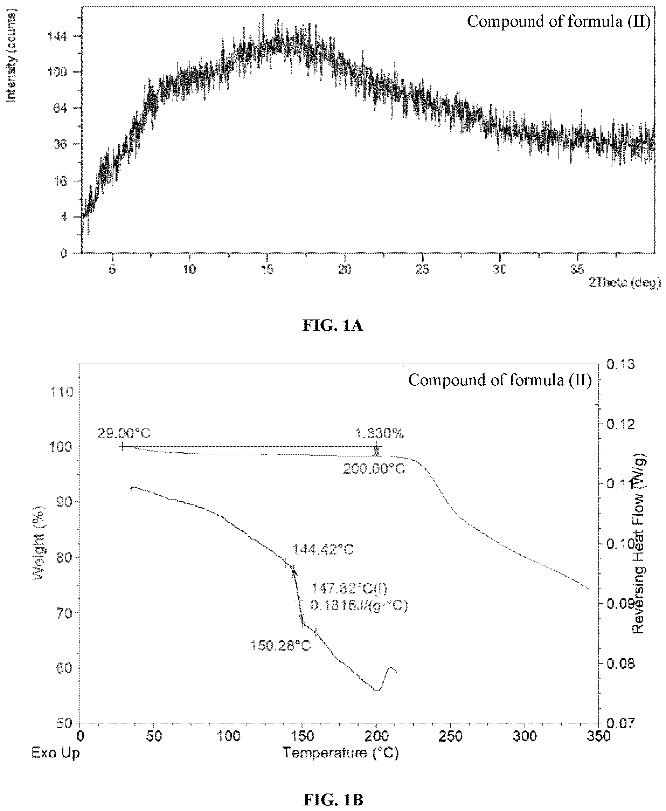

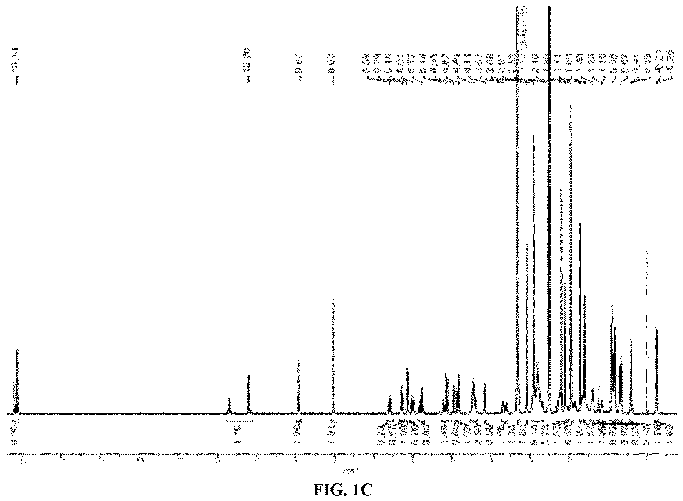

The structure of the exemplary compound is shown in formula (II) (hereinafter also referred to as TNP-2198), and the starting free sample was identified as an amorphous sample (FIG. 1A). The TGA/mDSC results (FIG. 1B) show a weight loss of 1.8% before 200 °C, with a glass transition temperature of 147.8 °C. ¹H NMR results (DMSO-*d₆*, FIG. 1C) show the consistency with the target substance provided.

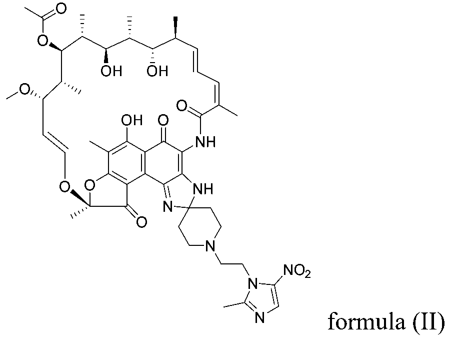

The approximate solubility of the starting free base of the exemplary compound of formula (II) in 5 single solvents and 9 mixed solvents at room temperature (about 25 °C) was also tested. The results are shown in Table 1.

**Table 1. Approximate solubility (mg/mL) of the starting free base at room temperature**

| **Solvent** | **Solubility** | **Solvent (v:v)** | **Solubility** | **Solvent (v:v)** | **Solubility** |
|---|---|---|---|---|---|
| IPA | S>39.2 | IPA:H₂O (19:1) | S>42.2 | 2-MeTHF:MTBE (1:1) | S>42.4 |
| EtOAc | S>40.8 | IPA:H₂O (9:1) | S>41.4 | EtOAc:MTBE (1:1) | S>41.4 |
| MTBE | 6.7<S<20.1 | IPA:H₂O (5:1) | S>41.2 | Toluene:MTBE (1:1) | S>42.4 |
| Toluene | S>41.4 | IPA:H₂O (3:1) | S>41.2 | IPA:n-Heptane (1:1) | 20.7<S<41.4 |
| 2-MeTHF | S>41.4 | IPA:H₂O (1:1) | 20.5<S<41.0 | -- | -- |

| | | | | | |
|---|---|---|---|---|---|
| Notes for Table 1: --: No data collection was conducted. | | | | | |

### (2) Preparation and characterization of salt forms of exemplary compound

35 mg of crude free base (92.64% purity) was added into an HPLC vial;
the acid ligands in the corresponding molar ratios in the table were added into the HPLC vial (the liquid acid was diluted with a solvent before the addition of the free base), and the corresponding solvent was added. The mixture was magnetically stirred for a moment at room temperature, the corresponding antisolvent in the table was added, and if necessary, a small amount of the corresponding seed crystal was added;
After stirring overnight, the sample was separated by centrifugation (10000 rpm, 2 min) and dried in vacuum at room temperature (25 °C) for 4 h. The solid was collected and characterized. The results are shown in Table 2.

**Table 2. Summary of preparation and characterization data**

| **Experiment No.** | **Ligand** | **Acid/base feeding ratio** | **Solvent (v:v)/volume (mL)** | **Antisolvent/volume (mL)** | **Spontaneous crystallization or not** | **Slurry state** | **Crystallinity** | **Appearance after drying** | **Chemical purity (area%)** | **Yield (%)** | **Acid/base molar ratio (¹H NMR/IC)** |
|---|---|---|---|---|---|---|---|---|---|---|---|
| 01 | Triphenylacetic acid | 1:1 | MTBE/0.5 | **-**- | Suspension | Good | Moderate | Powder | 93.64 | 86 | 1.1 |
| 02 | | | EtOAc/0.4 | MTBE/0.2 | Yes | Good | Moderate | Powder | 97.23 | 53 | 1.1 |
| 03 | | | EtOAc/0.4 | MTBE/0.4 | Yes | Good | Moderate | Powder | 97.32 | 63 | 1.1 |
| 04 | | | EtOAc/0.3 | -- | Yes | Good | Moderate | Powder | 95.37 | 77 | 1.1 |
| 05 | | | Toluene/0.2 | MTBE/0.6 | No | Good | Moderate | Powder | 93.32 | 69 | 1.0 |

| | | | | | | | | | | | |
|---|---|---|---|---|---|---|---|---|---|---|---|
| Notes for Table 2: The yield was an approximation based on the screened system and does not represent the final process system; --: No data collection was conducted. | | | | | | | | | | | |

### Example 2

### Comparative stability study of monotriphenylacetate salt of exemplary compound

(1) The monotriphenylacetate salt samples of the exemplary compound of formula (II) and the comparative examples (hydrochloride and phosphate salts of the exemplary compound) were subjected to HPLC characterization after vacuum drying at room temperature (about 25 °C) for 5 h. The results are shown in Table 3.

**Table 3. Summary of characterization data for different salt forms of exemplary compound**

| **Experiment No.** | **HPLC purity (area%) of compound of formula (II)** | **Ligand** | **Crystallinity** | **Appearance after drying** | **HPLC purity (area%)** | **Yield (%)** | **Acid/base molar ratio (¹H NMR/IC)** |
|---|---|---|---|---|---|---|---|
| 06 | 92.53 | Triphenylacetic acid | Moderate | Powder | 98.90 | 75 | 1.1 |
| 07 | 97.23 | Triphenylacetic acid | Moderate | Powder | 99.84 | 80 | 1.0 |
| 08 | 92.53 | Hydrochloric acid | Amorphous form | Powder | 91.06 | 85 | -- |
| 09 | 99.40 | Phosphoric acid | Low | Agglomerated solid | 95.18 | 86 | 1.1 |
| 10 | 99.40 | Hydrochloric acid | Amorphous form | Powder | 98.45 | 89 | 1.0 |

| | | | | | | | |
|---|---|---|---|---|---|---|---|
| Notes for Table 3: The yield was an approximation based on the screened system and does not represent the final process system; --: the purity/yield was relatively low, and the further acid/base molar ratio data were not collected; | | | | | | | |

As can be seen from the salt formation results in Table 3, in the salt formation process of the compound of formula II with different acids, the purity of the compound was reduced and degradation impurities were generated in the phosphate and hydrochloride salt formation process. In contrast, the monotriphenylacetate salt exhibited superior crystallinity and significantly improved purity of the compound, thereby achieving the purpose of separation and purification.
(2) Stability test

Samples were separately taken from two crystal forms I and II of the monotriphenylacetate salt of the exemplary compound of formula (II) and left to stand in open conditions at 25 °C/60% RH and 40 °C/75% RH for 3 days. The results are shown in Table 4.

The results in Table 4 show that in the 2 stability test conditions, the monotriphenylacetate samples did not change significantly in appearance and purity; with reference to the results in Tables 3 and 4, monotriphenylacetate salt exhibited moderate crystallinity as compared to the hydrochloride and phosphate salts, and the crystal form was unchanged in the 2 conditions, demonstrating that the monotriphenylacetate salt of the exemplary compound has good performance in terms of crystallinity and stability.

**Table 4. Summary of stability study data**

| **Starting sample** | | | **Conditions** | **Purity (Area%)** | **Change in crystal form** | **Change in appearance** |
|---|---|---|---|---|---|---|
| **Salt form** | **Experiment No.** | **Purity (Area%)** | | | | |
| Crystal form I of triphenylacetate salt | 02 (powder) | 97.23 | 25 °C/60% RH Open, 3 days | 98.09 | No change | No change |
| | | | 40 °C/75% RH Open, 3 days | 98.22 | No change | No change |
| Crystal form II of triphenylacetate salt | 01 (powder) | 93.64 | 25 °C/60% RH Open, 3 days | 93.29 | No change | No change |
| | | | 40 °C/75% RH Open, 3 days | 93.79 | No change | No change |

### Example 3

### Preparation of crystal forms I-IV of the present application

### (1) Preparation of crystal form I

1.00 g of compound TNP-2198 was added to 10 mL of ethyl acetate (EtOAc) solvent, and a solution of triphenylacetic acid in ethyl acetate (0.30 g + 2 mL of EtOAc) was slowly added dropwise. The mixture was stirred at 20-30 °C for 18-20 h, and a solid was precipitated. 3 mL of ethyl acetate was added to the reactant, and after filtration, the wet cake was washed with 2.5 mL of the solvent. The wet cake was dried in vacuum at 20-30 °C for more than 4 h to finally give a crystal form powder (0.83 g, 63.35% yield, 99.94% HPLC purity). A sample of the crystal form powder was taken for XRPD test, TGA test, and DSC test, and the results are shown in FIG. 2, Table 5, and FIGs. 3A and B.

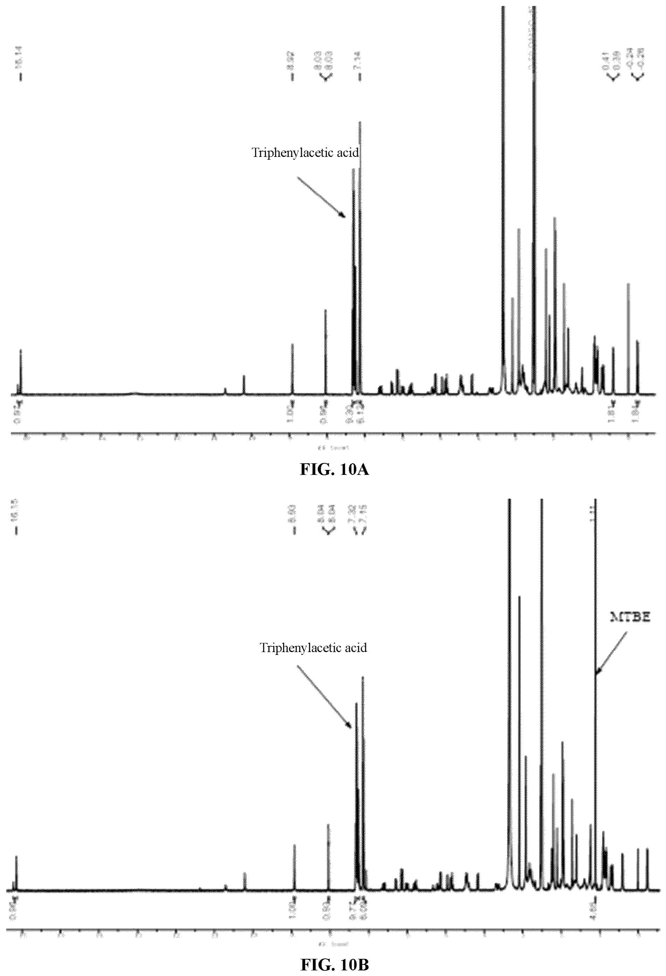

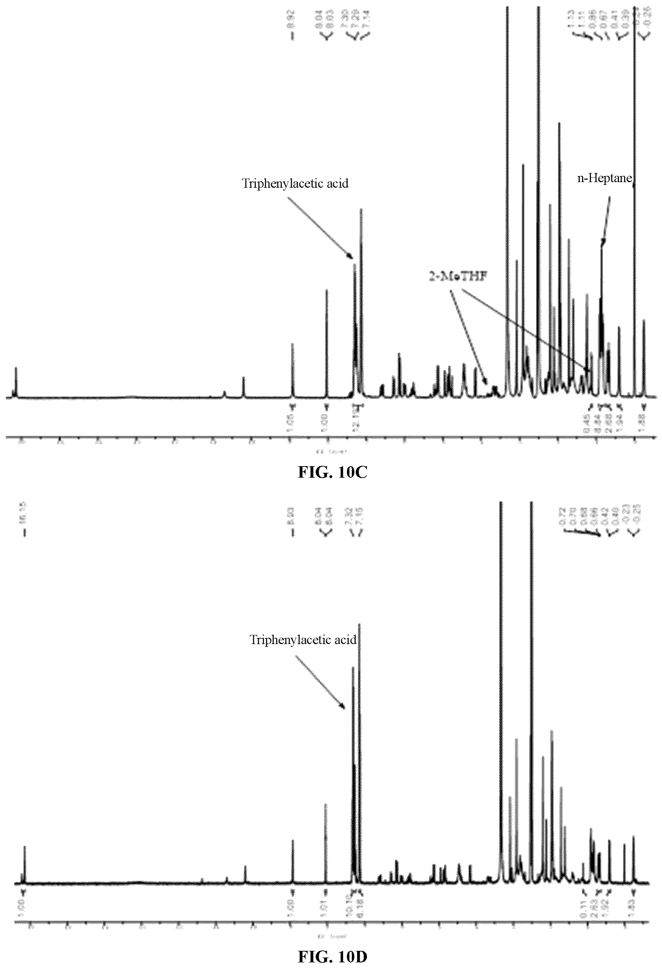

The characterization results for crystal form I: The diffraction peaks in XRPD demonstrated very high crystallinity of crystal form I. ¹H NMR (DMSO-*d₆*) suggested that the stoichiometric acid/base molar ratio was 1.0, and no solvent residue was observed. The TGA results showed a weight loss of 0.7% (~0.5 H₂O) before heating to 180 °C. The DSC results showed a starting temperature point of the weight loss at 166.5 °C. The crystal form I showed no change in appearance upon heating to 120 °C and the heated sample showed negligible weight loss prior to decomposition (FIGs 10A and B); upon heating to 180 °C, the melting of the crystal form I sample was observed. The above data indicate that crystal form I may be an anhydrate of the monotriphenylacetate salt.

Further purification method one for the crystal form I: 1.8 g of the crystal form I of TNP-2198 triphenylacetate (95.1% purity) was added into a reaction flask, followed by the addition of 18 mL (10 parts by volume) of 2-butanone. The mixture was heated to 50 °C, stirred for complete dissolution, and cooled to 25 °C. Then 18 mg (1%) of a seed crystal of the crystal form I of TNP-2198 triphenylacetate was added. The mixture was stirred at this temperature for 1 h, and then 36 mL (20 parts by volume) of n-heptane was slowly added dropwise. The mixture was stepwise cooled to 0 °C, stirred at this temperature for 11 h, and filtered to give a wet product (1.45 g). The wet product was added to a reaction flask, and 18 mL (10 parts by volume) of 2-butanone was added. The mixture was heated to 50 °C, stirred to dissolve the wet product completely, and cooled to 25 °C. Then 18 mg (1%) of a seed crystal of the crystal form I of TNP-2198 triphenylacetate was added. The mixture was stirred at this temperature for 2 h, and then 36 mL (20 parts by volume) of n-heptane was added dropwise. The mixture was stepwise cooled to 0 °C, stirred at this temperature for 14 h, filtered, and dried to give a product (1.35 g, 99.7% purity).

Further purification method two for the crystal form I: 1.8 g of the crystal form I of TNP-2198 triphenylacetate (95.1% purity) was added into a reaction flask, followed by the addition of 18 mL (10 parts by volume) of acetone. The mixture was heated to 50 °C, stirred for complete dissolution, and cooled to 25 °C. Then 18 mg (1%) of a seed crystal of the crystal form I of TNP-2198 triphenylacetate was added. The mixture was stirred at this temperature for 1 h, and then 36 mL (20 parts by volume) of n-heptane was slowly added dropwise. The mixture was stepwise cooled to 0 °C, stirred at this temperature for 11 h, and filtered to give a wet product (1.45 g). The wet product was added to a reaction flask, and 18 mL (10 parts by volume) of acetone was added. The mixture was heated to 50 °C, stirred to dissolve the wet product completely, and cooled to 25 °C. Then 18 mg (1%) of a seed crystal of the crystal form I of TNP-2198 triphenylacetate was added. The mixture was stirred at this temperature for 2 h, and then 36 mL (20 parts by volume) of n-heptane was added dropwise. The mixture was stepwise cooled to 0 °C, stirred at this temperature for 14 h, filtered, and dried to give a product (1.29 g, 99.0% purity).

**Table 5. X-ray powder diffraction data**

| Index | Angle | d Value | Net Intensity | Gross Intensity | Rel. Intensity | Position | Intensity |
|---|---|---|---|---|---|---|---|
| 1 | 4.684 ° | 18.84999 Å | 64.2 | 87.2 | 7.4% | 4.684 | 64.197 |
| 2 | 6.108 ° | 14.45737 Å | 864 | 909 | 100.0% | 6.108 | 863.545 |
| 3 | 9.147 ° | 9.66070 Å | 54.8 | 121 | 6.3% | 9.147 | 54.812 |
| 4 | 9.928 ° | 8.90235 Å | 355 | 432 | 41.1% | 9.928 | 355.144 |
| 5 | 11.488 ° | 7.69638 Å | 669 | 755 | 77.4% | 11.488 | 668.726 |
| 6 | 12.456 ° | 7.10066 Å | 207 | 291 | 24.0% | 12.456 | 206.966 |
| 7 | 14.410 ° | 6.14161 Å | 802 | 887 | 92.9% | 14.410 | 802.281 |
| 8 | 14.887 ° | 5.94604 Å | 168 | 257 | 19.5% | 14.887 | 168.246 |
| 9 | 16.154 ° | 5.48250 Å | 392 | 487 | 45.4% | 16.154 | 392.465 |
| 10 | 16.814 ° | 5.26871 Å | 126 | 221 | 14.6% | 16.814 | 126.133 |
| 11 | 17.832 ° | 4.97008 Å | 85.1 | 180 | 9.9% | 17.832 | 85.114 |
| 12 | 18.858 ° | 4.70195 Å | 523 | 626 | 60.5% | 18.858 | 522.706 |
| 13 | 19.310 ° | 4.59295 Å | 213 | 317 | 24.7% | 19.310 | 212.926 |
| 14 | 20.342 ° | 4.36225 Å | 216 | 319 | 25.0% | 20.342 | 216.312 |
| 15 | 20.772 ° | 4.27282 Å | 96.7 | 197 | 11.2% | 20.772 | 96.724 |
| 16 | 21.097 ° | 4.20773 Å | 94.0 | 191 | 10.9% | 21.097 | 93.958 |
| 17 | 21.881 ° | 4.05867 Å | 44.4 | 133 | 5.1% | 21.881 | 44.411 |
| 18 | 22.464 ° | 3.95475 Å | 48.9 | 132 | 5.7% | 22.464 | 48.874 |
| 19 | 23.253 ° | 3.82222 Å | 48.9 | 131 | 5.7% | 23.253 | 48.880 |
| 20 | 23.956 ° | 3.71167 Å | 101 | 183 | 11.7% | 23.956 | 101.248 |
| 21 | 24.347 ° | 3.65291 Å | 274 | 354 | 31.7% | 24.347 | 274.104 |
| 22 | 26.890 ° | 3.31299 Å | 33.9 | 101 | 3.9% | 26.890 | 33.934 |
| 23 | 28.804 ° | 3.09699 Å | 62.4 | 126 | 7.2% | 28.804 | 62.426 |
| 24 | 29.383 ° | 3.03733 Å | 32.6 | 96.4 | 3.8% | 29.383 | 32.628 |
| 25 | 30.911 ° | 2.89058 Å | 51.0 | 115 | 5.9% | 30.911 | 51.036 |
| 26 | 32.831 ° | 2.72573 Å | 19.5 | 80.0 | 2.3% | 32.831 | 19.477 |
| 27 | 35.144 ° | 2.55145 Å | 44.7 | 106 | 5.2% | 35.144 | 44.692 |

### (2) Preparation of crystal forms II-III

1.00 g of compound TNP-2198 was added to a 13 mL solution of methyl tert-butyl ether (MTBE), and a solution of triphenylacetic acid in methyl tert-butyl ether (0.30 g + 2 mL of MTBE) was slowly added dropwise. The mixture was stirred at 20-30 °C for 18-20 h, and a solid was precipitated. The mixture was filtered, and the wet cake was washed with 3 mL of the solvent. A crystal form II powder was obtained (0.83 g, 63.35% yield, 99.28% HPLC purity). A sample of the crystal form powder was taken for XRPD test, TGA test, and DSC test, and the results of the crystal form II are shown in FIG. 4, Table 6, and FIGs. 5A and B. The crystal form III was obtained by reactive crystallization of dried crystal form II in a mixed solvent of 2-methyltetrahydrofuran and n-heptane and drying in vacuum at 40 °C overnight. The results are shown in FIG. 6, Table 7, and FIGs. 7A and B.

Characterization results for crystal form II: ¹H NMR (DMSO-*d₆*) suggested a stoichiometric acid/base molar ratio of 1.1; The determination of the MTBE solvent content suggested a stoichiometric solvent/API ratio of 0.5 (about 3.6%). The TGA results showed a weight loss of 2.7% from room temperature to 130 °C, followed by a weight loss of 4.0% from 130 °C to 185 °C (MTBE + H₂O). The DSC results showed endothermic peaks at 61.9 °C, 146.8 °C, and 184.6 °C. The crystal form II showed no appearance change after being heated to 100 °C. After being heated to 120 °C, ¹H NMR (DMSO-*d₆*) suggested a stoichiometric MTBE/API ratio of 0.2 (about 1.7%) in the crystal form II.

Characterization results for crystal form III: ¹H NMR (DMSO-*d₆*) suggested a stoichiometric acid/base molar ratio of 0.8. The determination of the solvent content suggested a stoichiometric 2-MeTHF/API ratio of 0.2 (about 0.9%) and a stoichiometric n-heptane/API ratio of 0.4 (about 2.9%). The TGA results showed a weight loss of 5.7% (solvent + H₂O) before heating to 180 °C. The DSC results showed endothermic peaks at 64.7 °C, 142.8 °C, 159.4 °C, and 177.0 °C.

All of the above data indicate that the crystal forms II and III are likely a hydrate or a solvate of the triphenylacetate salt.

**Table 6. X-ray powder diffraction data**

| Index | Angle | d Value | Net Intensity | Gross Intensity | Rel. Intensity | Position | Intensity |
|---|---|---|---|---|---|---|---|
| 1 | 3.998° | 22.08240 Å | 12.2 | 24.4 | 7.0% | 3.998 | 12.237 |
| 2 | 4.831 ° | 18.27653 Å | 68.3 | 90.4 | 39.0% | 4.831 | 68.316 |
| 3 | 5.613 ° | 15.73250 Å | 76.3 | 106 | 43.6% | 5.613 | 76.312 |
| 4 | 6.852 ° | 12.88950 Å | 55.1 | 94.5 | 31.5% | 6.852 | 55.120 |
| 5 | 7.995 ° | 11.04992 Å | 34.7 | 79.6 | 19.8% | 7.995 | 34.684 |
| 6 | 8.505 ° | 10.38865 Å | 57.9 | 104 | 33.1% | 8.505 | 57.871 |
| 7 | 9.372 ° | 9.42921 Å | 44.2 | 93.0 | 25.2% | 9.372 | 44.156 |
| 8 | 9.775 ° | 9.04147 Å | 42.2 | 92.8 | 24.1% | 9.775 | 42.200 |
| 9 | 10.163 ° | 8.69667 Å | 56.6 | 108 | 32.3% | 10.163 | 56.586 |
| 10 | 11.372 ° | 7.77477 Å | 175 | 228 | 100.0% | 11.372 | 175.077 |
| 11 | 12.062 ° | 7.33175 Å | 141 | 193 | 80.7% | 12.062 | 141.293 |
| 12 | 13.929 ° | 6.35285 Å | 37.6 | 87.4 | 21.5% | 13.929 | 37.641 |
| 13 | 14.540 ° | 6.08700 Å | 66.8 | 118 | 38.1% | 14.540 | 66.768 |
| 14 | 15.437 ° | 5.73530 Å | 21.9 | 74.7 | 12.5% | 15.437 | 21.895 |
| 15 | 16.103 ° | 5.49962 Å | 172 | 225 | 98.2% | 16.103 | 171.849 |
| 16 | 17.014 ° | 5.20731 Å | 65.0 | 119 | 37.1% | 17.014 | 65.017 |
| 17 | 17.220 ° | 5.14546 Å | 43.2 | 97.2 | 24.7% | 17.220 | 43.192 |
| 18 | 18.142 ° | 4.88586 Å | 154 | 210 | 88.2% | 18.142 | 154.347 |
| 19 | 19.344 ° | 4.58489 Å | 52.2 | 110 | 29.8% | 19.344 | 52.176 |
| 20 | 19.797 ° | 4.48110 Å | 135 | 192 | 77.1% | 19.797 | 135.001 |
| 21 | 20.581 ° | 4.31206 Å | 33.7 | 88.9 | 19.2% | 20.581 | 33.652 |
| 22 | 21.110 ° | 4.20527 Å | 44.9 | 97.5 | 25.7% | 21.110 | 44.948 |
| 23 | 24.357 ° | 3.65151 Å | 123 | 169 | 70.1% | 24.357 | 122.810 |

**Table 7. X-ray powder diffraction data**

| Index | Angle | d Value | Net Intensity | Gross Intensity | Rel. Intensity | Position | Intensity |
|---|---|---|---|---|---|---|---|
| 1 | 4.102 ° | 21.52179 Å | 308 | 359 | 26.5% | 4.102 | 307.565 |
| 2 | 5.004 ° | 17.64440 Å | 307 | 393 | 26.4% | 5.004 | 306.939 |
| 3 | 5.758 ° | 15.33708 Å | 366 | 477 | 31.5% | 5.758 | 366.134 |
| 4 | 7.010 ° | 12.60055 Å | 130 | 279 | 11.2% | 7.010 | 130.282 |
| 5 | 8.161° | 10.82460 Å | 120 | 309 | 10.3% | 8.161 | 119.614 |
| 6 | 8.606 ° | 10.26698 Å | 229 | 436 | 19.7% | 8.606 | 229.167 |
| 7 | 9.571 ° | 9.23365 Å | 556 | 795 | 47.9% | 9.571 | 555.737 |
| 8 | 10.251 ° | 8.62244 Å | 149 | 406 | 12.8% | 10.251 | 148.653 |
| 9 | 11.488 ° | 7.69651 Å | 583 | 864 | 50.2% | 11.488 | 582.736 |
| 10 | 12.139 ° | 7.28498 Å | 495 | 784 | 42.7% | 12.139 | 495.282 |
| 11 | 14.103 ° | 6.27465 Å | 95.7 | 427 | 8.2% | 14.103 | 95.722 |
| 12 | 14.727 ° | 6.01028 Å | 183 | 532 | 15.8% | 14.727 | 183.202 |
| 13 | 16.184 ° | 5.47219 Å | 520 | 899 | 44.8% | 16.184 | 520.163 |
| 14 | 17.125 ° | 5.17364 Å | 294 | 684 | 25.3% | 17.125 | 294.120 |
| 15 | 18.259 ° | 4.85481 Å | 1161 | 1554 | 100.0% | 18.259 | 1160.755 |
| 16 | 19.152 ° | 4.63054 Å | 422 | 810 | 36.4% | 19.152 | 421.953 |
| 17 | 19.614 ° | 4.52243 Å | 795 | 1178 | 68.5% | 19.614 | 794.945 |
| 18 | 19.921 ° | 4.45349 Å | 737 | 1116 | 63.5% | 19.921 | 736.568 |
| 19 | 20.813 ° | 4.26442 Å | 471 | 834 | 40.6% | 20.813 | 471.143 |
| 20 | 21.599 ° | 4.11112 Å | 333 | 676 | 28.6% | 21.599 | 332.515 |
| 21 | 21.995° | 4.03792 Å | 173 | 504 | 14.9% | 21.995 | 172.749 |
| 22 | 23.061 ° | 3.85360 Å | 120 | 429 | 10.4% | 23.061 | 120.467 |
| 23 | 23.769 ° | 3.74043 Å | 221 | 520 | 19.0% | 23.769 | 220.865 |
| 24 | 24.523 ° | 3.62703 Å | 337 | 623 | 29.0% | 24.523 | 336.825 |
| 25 | 27.674° | 3.22086 Å | 67.0 | 296 | 5.8% | 27.674 | 67.010 |
| 26 | 28.704 ° | 3.10759 Å | 97.4 | 318 | 8.4% | 28.704 | 97.392 |
| 27 | 29.426 ° | 3.03290 Å | 88.0 | 298 | 7.6% | 29.426 | 88.030 |
| 28 | 31.275 ° | 2.85775 Å | 102 | 283 | 8.8% | 31.275 | 102.407 |
| 29 | 31.806 ° | 2.81122 Å | 93.8 | 273 | 8.1% | 31.806 | 93.768 |
| 30 | 32.815 ° | 2.72703 Å | 52.7 | 231 | 4.5% | 32.815 | 52.719 |
| 31 | 37.343 ° | 2.40613 Å | 85.4 | 244 | 7.4% | 37.343 | 85.359 |

### (3) Preparation of crystal form IV

The crystal form obtained in (2) was heated to 160 °C and then cooled to room temperature (about 25 °C) with N₂ purge to give the crystal form IV of the triphenylacetate salt with relatively low crystallinity. ¹H NMR (DMSO-*d₆*) suggested that the stoichiometric acid/base molar ratio was 1.1, and the residual amount of the MTBE solvent was negligible. Samples were taken for XRPD, TGA, and DSC tests, and the results are shown in FIG. 8, Table 8, and FIGs. 9A and B. The sample exhibited a weight loss of 2.0% (~1.5 H₂O) before 180 °C and an endothermic peak at 182.8 °C, as determined by TGA/DSC. The above data indicate that the crystal form IV may be a hydrate or an anhydrous form of the triphenylacetate salt.

**Table 8. X-ray powder diffraction data**

| Index | Angle | d Value | Net Intensity | Gross Intensity | Rel. Intensity | Position | Intensity |
|---|---|---|---|---|---|---|---|
| 1 | 4.850 ° | 18.20371 Å | 24.5 | 43.8 | 11.4% | 4.850 | 24.472 |
| 2 | 7.205 ° | 12.25975 Å | 40.2 | 86.9 | 18.7% | 7.205 | 40.226 |
| 3 | 10.640 ° | 8.30759 Å | 215 | 288 | 100.0% | 10.640 | 214.762 |
| 4 | 11.537 ° | 7.66370 Å | 74.7 | 151 | 34.8% | 11.537 | 74.684 |
| 5 | 14.637 ° | 6.04716 Å | 67.7 | 156 | 31.5% | 14.637 | 67.700 |
| 6 | 16.062 ° | 5.51368 Å | 140 | 232 | 65.1% | 16.062 | 139.834 |
| 7 | 18.083 ° | 4.90176 Å | 72.1 | 163 | 33.6% | 18.083 | 72.099 |
| 8 | 18.824 ° | 4.71037 Å | 35.5 | 125 | 16.5% | 18.824 | 35.511 |
| 9 | 20.088 ° | 4.41666 Å | 61.8 | 146 | 28.8% | 20.088 | 61.797 |
| 10 | 23.888 ° | 3.72199 Å | 65.3 | 131 | 30.4% | 23.888 | 65.297 |
| 11 | 24.261 ° | 3.66564 Å | 60.7 | 125 | 28.3% | 24.261 | 60.692 |
| 12 | 30.239 ° | 2.95327 Å | 17.9 | 63.9 | 8.3% | 30.239 | 17.902 |

### Example 4

### Evaluation of crystal form I

### (1) Hygroscopicity test (dynamic vapor sorption test, or DVS)

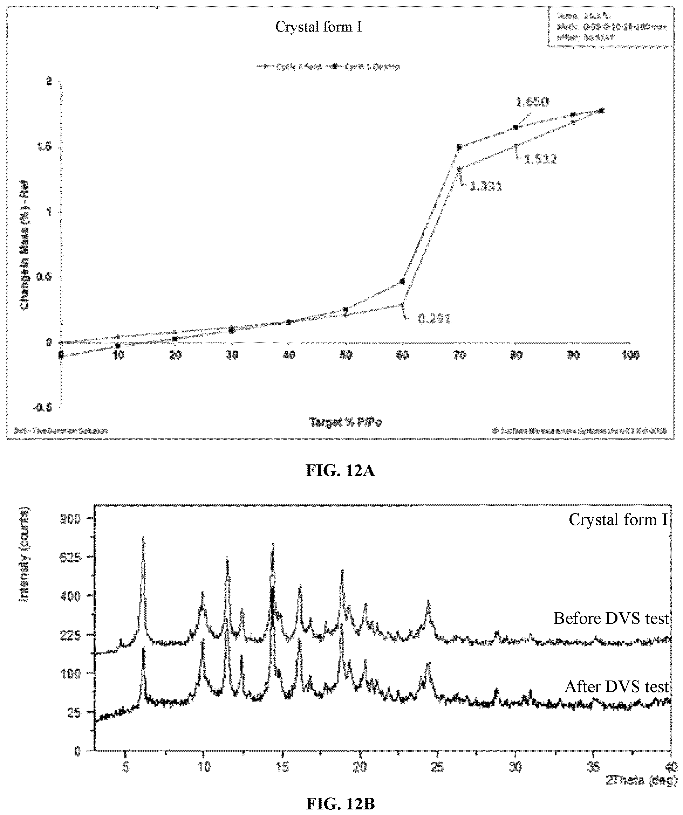

The DVS results of the crystal form I (FIG. 12A) show slow weight gains from 0 to 60% RH and 70% RH to 95% RH, and a rapid weight gain from 60% RH to 70% RH. A water absorption of 1.5% (about 1 H ₂ O) was observed at 25 °C/80% RH (relative to 0% RH), indicating that the crystal form I is slightly hygroscopic. The XRPD results (FIG. 12B) show that there was no change in appearance of this crystal form before/after DVS evaluation.

### (2) Kinetic solubility

About 10 mg/mL of sample (on a free base basis) was added to various biological media. After shaking at 100 rpm at 37 °C (for 1/4/24 h), samples 01-05 were centrifuged, and the solids were collected for XRPD test, while the supernatant was used for detection and pH measurement.

It can be observed that after 24 h, the solubility of the crystal form I in FaSSGF (about 1.6 mg/mL) was higher than those in the other four media, and no appearance changes were found in all media (FIG. 13, where the peak value at 31.6° corresponds to the solvent of NaCl).

**Table 9. Kinetic solubility of crystal form I in water and four biological media at 37 °C (mg/mL)**

| **Medium** | **1 h** | | | **4 h** | | | **24 h** | | |
|---|---|---|---|---|---|---|---|---|---|
| | S | pH | FC | S | pH | FC | S | pH | FC |
| Water | <LOQ | 4.45 | No | <LOQ | 4.44 | No | <LOQ | 4.62 | No |
| FaSSGF (1.6) | 1.3 | 1.55 | No | 1.6 | 1.57 | No | 1.6 | 1.54 | No |
| FeSSGF (5.0) | 0.12 | 5.03 | No | 0.18 | 5.07 | No | 0.22 | 5.07 | No |
| FeSSIF (5.0) | 0.12 | 4.85 | No | 0.12 | 4.84 | No | 0.14 | 4.84 | No |
| FaSSIF (6.5) | 0.012 | 6.37 | No | 0.0057 | 6.37 | No | 0.0075 | 6.40 | No |

| | | | | | | | | | |
|---|---|---|---|---|---|---|---|---|---|
| Notes for Table 9: Biological media: FaSSGF (fasted state simulated gastric fluid)/FeSSGF (fed state simulated gastric fluid)/FaSSIF (fasted state simulated intestinal fluid)/FeSSIF (fed state simulated intestinal fluid) S: Solubility (mg/mL), on a free radical basis; FC: change in appearance; LOQ = 0.099 µg/mL. | | | | | | | | | |

### Example 5

### Preparation of drugs using crystal form I of the present application

Preparation of crystal form I: A solution of a crude TNP-2198 product in ethyl acetate (92% purity, 72 kg) was concentrated by azeotropic drying to remove water, and then triphenylacetic acid (22.4 kg, 1 eq in total) was added in portions. The mixture was stirred at 25 °C for 3 h, cooled to 5 °C, successively stirred for 2 h, and filtered to give the crystal form I of TNP-2198 triphenylacetate.

Preparation of drug: The wet product obtained by the aforementioned process was added into a reaction kettle, and 385.2 kg of 2-butanone was added. The mixture was heated to 45 °C or higher, stirred for complete dissolution, and cooled to 20-30 °C. 72 g of a seed crystal of the crystal form I of TNP-2198 triphenylacetate was added. The mixture was stirred at 25 °C for 2 h, and then 655.3 kg of n-heptane was added dropwise. The mixture was slowly cooled to 5 °C, successively stirred for 2 h, and then filtered. The wet product was dried at 40 °C to give 66.23 kg of product (98.7% purity). The obtained product could be again recrystallized in a 2-butanone/n-heptane system to give the crystal form I of TNP-2198 triphenylacetate (99.0% purity or higher). The crystal form I of TNP-2198 triphenylacetate (51.5 kg) obtained in the previous step was dissolved in 1582 kg of dichloromethane at 20-30 °C, and 956 kg of 1% diluted Na₂CO₃ solution was added for washing and desalting. The solvent was replaced by ethyl acetate (until dichloromethane was less than 10%) through distillative solvent exchange, and the above solution was added dropwise to 1434 kg of n-heptane for precipitation. The precipitate was collected by filtration and dried at 45 °C to give free amorphous TNP-2198 (37.2 kg, 99.7% purity).

### Example 6

### Preparation and characterization of crystal forms V and VI of the present application

100.04 mg of the compound of formula (I-II) (hereinafter also referred to as rifabutin, 96.7% purity) was added to 1.5 mL of ethyl acetate (EtOAc) solution and stirred until complete dissolution. 34.01 mg of triphenylacetic acid was added, and the mixture was stirred at 20-30 °C for 18 h. n-Heptane (1.5 mL) was added into the reaction system described above, and the mixture was filtered. The wet cake was washed with n-heptane (2 mL) and dried in vacuum at 20-30 °C for 8-16 h to give a product of the crystal form V (65.11 mg, 99.5% HPLC purity).

100.01 mg of rifabutin was added to 1.5 mL of methyl tert-butyl ether (MTBE) solution, and the mixture was stirred until complete dissolution. 34.03 mg of triphenylacetic acid was added, and the mixture was stirred at 20-30 °C for 18 h. The mixture obtained in the above process was filtered. The wet cake was washed with MTBE (2 mL) and dried in vacuum at 20-30 °C for 8-16 h to give a product of the crystal form VI (54.81 mg, 99.3% HPLC purity).

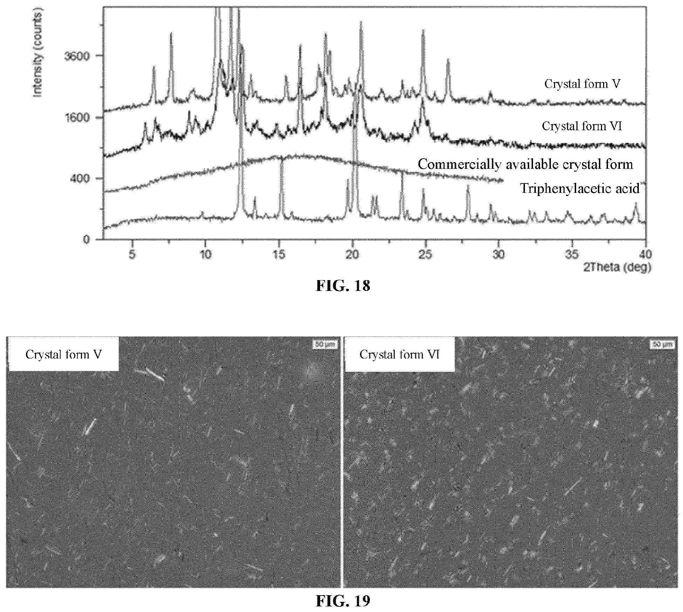

Samples of the crystal forms V and VI were taken for XRPD test, TGA test, and DSC test. The results of the crystal form V are shown in FIG. 14, Table 10, and FIGs. 15A and B, and the results of the crystal form VI are shown in FIG. 16, Table 11, and FIGs. 17A and B, which were compared with a commercially available amorphous product of rifabutin as shown in FIG. 18. It can be observed that the crystal forms V and VI were substantially identical, and the crystal form V had better crystallinity.

**Table 10. X-ray powder diffraction data**

| Index | Angle | d Value | Net Intensity | Gross Intensity | Rel. Intensity | Position | Intensity |
|---|---|---|---|---|---|---|---|
| 1 | 6.454 ° | 13.68428 Å | 395 | 480 | 7.5% | 6.454 | 395.163 |
| 2 | 7.645 ° | 11.55531 Å | 807 | 908 | 15.3% | 7.645 | 806.903 |
| 3 | 9.096 ° | 9.71458 Å | 95.8 | 214 | 1.8% | 9.096 | 95.758 |
| 4 | 10.840 ° | 8.15541 Å | 5262 | 5405 | 100.0% | 10.840 | 5261.507 |
| 5 | 11.714 ° | 7.54850 Å | 1606 | 1754 | 30.5% | 11.714 | 1606.306 |
| 6 | 12.255 ° | 7.21663 Å | 1245 | 1390 | 23.7% | 12.255 | 1244.687 |
| 7 | 12.621 ° | 7.00819 Å | 48.1 | 190 | 0.9% | 12.621 | 48.087 |
| 8 | 13.080 ° | 6.76339 Å | 251 | 385 | 4.8% | 13.080 | 251.222 |
| 9 | 13.387 ° | 6.60874 Å | 67.8 | 195 | 1.3% | 13.387 | 67.762 |
| 10 | 15.492 ° | 5.71501 Å | 221 | 343 | 4.2% | 15.492 | 221.464 |
| 11 | 16.440 ° | 5.38758 Å | 598 | 733 | 11.4% | 16.440 | 597.633 |
| 12 | 17.770 ° | 4.98740 Å | 291 | 447 | 5.5% | 17.770 | 290.534 |
| 13 | 18.183 ° | 4.87508 Å | 726 | 887 | 13.8% | 18.183 | 725.863 |
| 14 | 18.466 ° | 4.80077 Å | 512 | 674 | 9.7% | 18.466 | 511.827 |
| 15 | 18.858 ° | 4.70199 Å | 82.3 | 245 | 1.6% | 18.858 | 82.316 |
| 16 | 19.479 ° | 4.55335 Å | 105 | 264 | 2.0% | 19.479 | 104.653 |
| 17 | 19.775 ° | 4.48592 Å | 189 | 347 | 3.6% | 19.775 | 189.426 |
| 18 | 20.601 ° | 4.30793 Å | 925 | 1071 | 17.6% | 20.601 | 924.995 |
| 19 | 21.980 ° | 4.04068 Å | 80.9 | 207 | 1.5% | 21.980 | 80.937 |
| 20 | 23.396 ° | 3.79913 Å | 183 | 314 | 3.5% | 23.396 | 183.364 |
| 21 | 24.093 ° | 3.69090 Å | 113 | 249 | 2.1% | 24.093 | 113.034 |
| 22 | 24.830 ° | 3.58298 Å | 818 | 951 | 15.5% | 24.830 | 818.035 |
| 23 | 25.624 ° | 3.47374 Å | 71.3 | 192 | 1.4% | 25.624 | 71.271 |
| 24 | 26.543 ° | 3.35542 Å | 458 | 572 | 8.7% | 26.543 | 458.044 |
| 25 | 29.434 ° | 3.03213 Å | 103 | 197 | 2.0% | 29.434 | 103.317 |

**Table 11. X-ray powder diffraction data**

| Index | Angle | d Value | Net Intensity | Gross Intensity | Rel. Intensity | Position | Intensity |
|---|---|---|---|---|---|---|---|
| 1 | 5.887 ° | 14.99965 Å | 165 | 243 | 18.8% | 5.887 | 164.620 |
| 2 | 6.627 ° | 13.32748 Å | 135 | 233 | 15.5% | 6.627 | 135.419 |
| 3 | 7.541° | 11.71424 Å | 69.6 | 186 | 8.0% | 7.541 | 69.645 |
| 4 | 8.866 ° | 9.96642 Å | 212 | 351 | 24.3% | 8.866 | 212.219 |
| 5 | 9.306 ° | 9.49615 Å | 133 | 280 | 15.3% | 9.306 | 133.449 |
| 6 | 10.120 ° | 8.73359 Å | 113 | 270 | 12.9% | 10.120 | 112.936 |
| 7 | 11.045 ° | 8.00393 Å | 731 | 894 | 83.7% | 11.045 | 731.086 |
| 8 | 11.761 ° | 7.51865 Å | 417 | 581 | 47.8% | 11.761 | 417.495 |
| 9 | 12.448 ° | 7.10496 Å | 874 | 1035 | 100.0% | 12.448 | 873.617 |
| 10 | 13.458 ° | 6.57417 Å | 115 | 267 | 13.1% | 13.458 | 114.634 |
| 11 | 14.831 ° | 5.96835 Å | 89.0 | 233 | 10.2% | 14.831 | 88.980 |
| 12 | 15.635 ° | 5.66334 Å | 67.2 | 218 | 7.7% | 15.635 | 67.237 |
| 13 | 16.449 ° | 5.38485 Å | 499 | 662 | 57.1% | 16.449 | 498.970 |
| 14 | 17.352 ° | 5.10637 Å | 96.3 | 268 | 11.0% | 17.352 | 96.288 |
| 15 | 17.866 ° | 4.96074 Å | 169 | 343 | 19.3% | 17.866 | 168.882 |
| 16 | 18.146 ° | 4.88492 Å | 490 | 665 | 56.1% | 18.146 | 490.320 |
| 17 | 19.998 ° | 4.43646 Å | 185 | 351 | 21.1% | 19.998 | 184.563 |
| 18 | 20.585 ° | 4.31124 Å | 453 | 612 | 51.8% | 20.585 | 452.678 |
| 19 | 21.874 ° | 4.05995 Å | 75.5 | 210 | 8.6% | 21.874 | 75.519 |
| 20 | 24.261 ° | 3.66562 Å | 139 | 270 | 15.9% | 24.261 | 138.780 |
| 21 | 24.774 ° | 3.59086 Å | 341 | 470 | 39.0% | 24.774 | 340.692 |
| 22 | 25.137 ° | 3.53992 Å | 107 | 234 | 12.3% | 25.137 | 107.076 |
| 23 | 29.467 ° | 3.02882 Å | 28.3 | 120 | 3.2% | 29.467 | 28.322 |
| 24 | 30.125 ° | 2.96411 Å | 31.3 | 118 | 3.6% | 30.125 | 31.327 |
| 25 | 32.393 ° | 2.76158 Å | 29.0 | 102 | 3.3% | 32.393 | 28.957 |

### Example 7

### Study on stability of crystal forms V and VI of the present application

The commercially available amorphous form of rifabutin and the crystal forms V and VI of the present application were separately placed in a constant-temperature and constant-humidity incubator at 40 °C and 75% humidity for acceleration test. After one month, the change in purity was determined. The determination results show that the purity of the amorphous product of rifabutin was reduced from 95.9% of the original sample to 95.54%, the purity of the crystal form V was reduced from 99.34% of the original sample to 99.07%, and the purity of the crystal form VI was reduced from 99.1% to 98.87%. It can be seen that the crystal forms V and VI of the present application have comparable or even higher stability as compared with the commercially available amorphous products.

### Example 8

### Study on crystallization and purification ability of crystal form V of the present application

In this example, the crystal system of rifabutin triphenylacetate (in solvents of tetrahydrofuran/n-heptane, butanone/n-heptane, and ethyl acetate/n-heptane) was investigated by taking a crude product of amorphous rifabutin as a starting material, and the crystalline rifabutin in Examples 1 and 2 of Patent No. CN103408571B was repeated as a control. The purity and yield were collected, and the results are shown in Table 12.
(1) Comparison with crystalline rifabutin:
   No. 2: 0.50 g of amorphous rifabutin was added to 35 mL of n-hexane while stirring, and heated and stirred at reflux for 30 min before the heating was stopped. The mixture was cooled to 0 °C at a cooling rate of 10 °C/h and filtered to give 0.40 g of crystalline rifabutin (95.46% purity). Compared with the starting material (No. 1), substantially no purification effects were observed.
   No. 3: 0.50 g of amorphous rifabutin was added to 10 mL of a 19:1 (v:v) mixed solvent of n-hexane and ethyl acetate while stirring, and heated and stirred at reflux for 30 min before the heating was stopped. The mixture was cooled to 0 °C at a cooling rate of 10 °C/h and filtered to give 0.22g of crystalline rifabutin (95.98% purity). Compared with the starting purity, substantially no purification effects were observed.
(2) The crystal forms V and VI of the present application:
   Preparation of crystal form V (No. 4): 7.89 g of amorphous rifabutin was added to 40 mL of ethyl acetate, and the mixture was stirred for complete dissolution. 2.68 g of triphenylacetic acid and 40 mL of n-heptane were sequentially added. The reaction solution was stirred at room temperature for 4 h and filtered. The wet product was dried in vacuum to constant weight to give the crystal form V of rifabutin triphenylacetate (7.75 g, 99.3% purity).
   Preparation of crystal form V (No. 5): 8.01g of amorphous rifabutin was added to 80 mL of methyl tert-butyl ether, and the mixture was stirred for complete dissolution. 2.70 g of triphenylacetic acid was added. The reaction solution was stirred at room temperature for 4 h and filtered. The wet product was dried in vacuum to constant weight to give the crystal form VI of rifabutin triphenylacetate (7.10g, 99.2% purity).

The above results show that compared with the crystal purification in Examples 1 and 2 of Patent No. CN103408571B, the crystal form V exhibited a greatly improved purity on the basis of the original crude product of rifabutin along with a high yield, and such a purification effect was not achieved in the crystal forms of rifabutin disclosed in the prior art.

**Table 12. Crystal purification capacities of different crystal forms**

| **No.** | **Experimental subjects** | **Solvent** | **Purity** |
|---|---|---|---|
| 1 | Amorphous free base | - | 95.9% |
| 2 | Comparative crystal form 1 | ¹n-Hexane | 95.46% |
| 3 | Comparative crystal form 2 | ²n-Hexane and ethyl acetate | 95.98% |
| 4 | Crystal form V | Ethyl acetate and n-heptane | 99.3% |
| 5 | Crystal form VI | Methyl tert-butyl ether (MTBE) | 99.2% |

| | | | |
|---|---|---|---|
| Notes for Table 12: ¹: see Example 1 of Patent No. CN103408571B (Chengdu Fengqiao) for the method for preparing crystalline rifabutin; ²: see Example 2 of Patent No. CN103408571B (Chengdu Fengqiao) for the method for preparing crystalline rifabutin. | | | |

## Claims

1. A triphenylacetate salt of the compound of formula (I), or a hydrate, solvate, prodrug, metabolite or deuteride thereof: 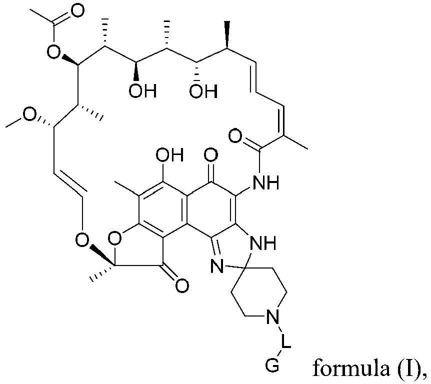 wherein L is a bond, alkyl, or the structure shown in 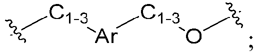 Ar is optionally substituted aryl; G is hydrogen, alkyl, haloalkyl, alkenyl, alkynyl, optionally substituted cycloalkyl, or optionally substituted heterocyclyl.

2. The triphenylacetate salt according to claim 1, wherein the molar ratio of the compound of formula (I) to triphenylacetic acid is 0.8-1.2.

3. The triphenylacetate salt according to claim 1 or 2, wherein the salt is a monotriphenylacetate salt.

4. The triphenylacetate salt according to any one of claims 1-3, wherein L is C₁-C₆ alkyl.

5. The triphenylacetate salt according to any one of claims 1-3, wherein the structure of L is:

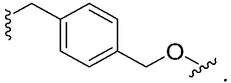

6. The triphenylacetate salt according to any one of claims 1-5, wherein G is C₁-C₆ alkyl, C₂-C₆ alkenyl, or C₂-C₆ alkynyl.

7. The triphenylacetate salt according to any one of claims 1-5, wherein G is C₁-C₃ monohaloalkyl or C₁-C₃ dihaloalkyl.

8. The triphenylacetate salt according to any one of claims 1-5, wherein G is optionally substituted C₃-C₈ cycloalkyl, an optionally substituted 3- to 8-membered heteroatom-containing monocyclic substituent, or an optionally substituted 4- to 12-membered heteroatom-containing bicyclic substituent.

9. The triphenylacetate salt according to any one of claims 1-8, wherein G is any one of the following structures:

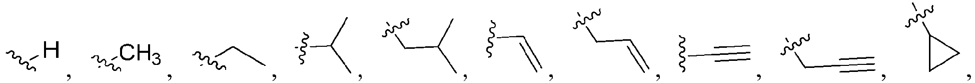

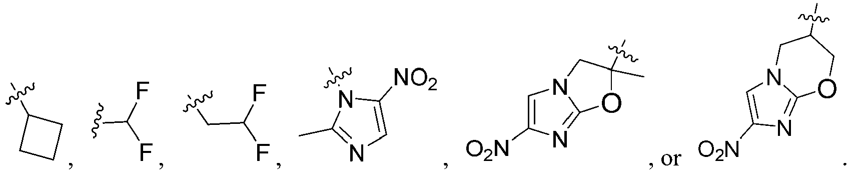

10. The triphenylacetate salt according to any one of claims 1-9, wherein the optionally substituted substituent is selected from: halogen, hydroxyl, carboxyl, carbonyl, amino, nitro, thiol, cyano, alkyl, alkenyl, alkynyl, cycloalkyl, aryl, heteroaryl, alkylamino, alkoxy, and carbonyl ester group.

11. The triphenylacetate salt according to any one of claims 1-10, wherein the structure of 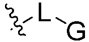 is any one of the following structures:

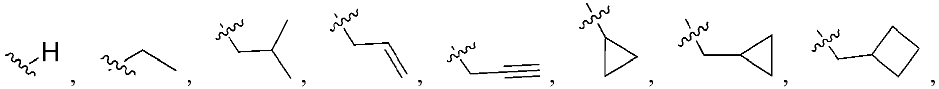

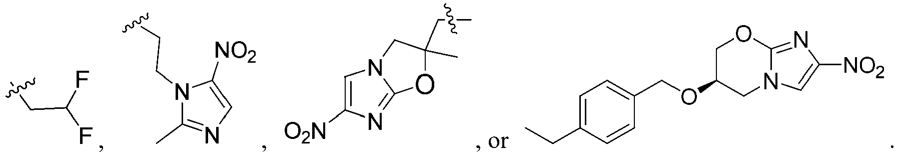

12. The triphenylacetate salt according to any one of claims 1-11, wherein the salt is a crystal, and the crystal is crystal form I formed by crystallizing the salt of formula (I-I): 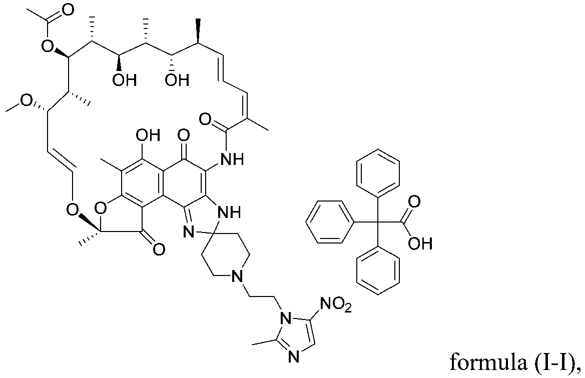 the crystal form I has an X-ray powder diffraction pattern comprising characteristic peaks at diffraction angles 20 of 6.11±0.2°, 9.93±0.2°, 11.49±0.2°, 14.41±0.2°, 16.15±0.2°, and 18.86±0.2°.

13. The triphenylacetate salt according to claim 12, wherein the crystal form I has an X-ray powder diffraction pattern comprising characteristic peaks at one or more diffraction angles 20 of 12.46±0.2°, 14.89±0.2°, 16.81±0.2°, 19.31±0.2°, 20.34±0.2°, and 24.35±0.2°.

14. The triphenylacetate salt according to claim 12 or 13, wherein the crystal form I has an X-ray powder diffraction pattern comprising characteristic peaks at diffraction angles 20 of 12.46±0.2°, 14.89±0.2°, 16.81±0.2°, 19.31±0.2°, 20.34±0.2°, and 24.35±0.2°.

15. The triphenylacetate salt according to any one of claims 12-14, wherein the crystal form I has an X-ray powder diffraction pattern substantially consistent with FIG. 2.

16. The triphenylacetate salt according to any one of claims 12-15, wherein the crystal form I is an anhydrate.

17. The triphenylacetate salt according to any one of claims 12-16, wherein the crystal form I has a differential scanning calorimetry profile comprising an endothermic peak at 174.2 °C.

18. The triphenylacetate salt according to any one of claims 12-17, wherein the crystal form I has a differential scanning calorimetry profile substantially consistent with FIG. 3B.

19. The triphenylacetate salt according to any one of claims 1-11, wherein the salt is a crystal, and the crystal is crystal form II formed by crystallizing the salt of formula (I-I): 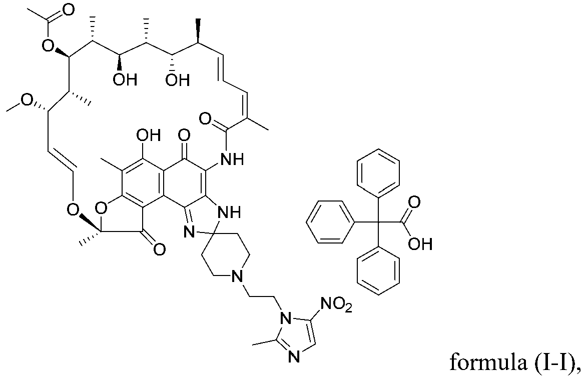 the crystal form II has an X-ray powder diffraction pattern comprising characteristic peaks at diffraction angles 20 of 11.37±0.2°, 12.06±0.2°, 16.10±0.2°, 18.14±0.2°, 19.80±0.2°, and 24.36±0.2°.

20. The triphenylacetate salt according to claim 19, wherein the crystal form II has an X-ray powder diffraction pattern comprising characteristic peaks at one or more diffraction angles 20 of 4.83±0.2°, 5.61±0.2°, 6.85±0.2°, 8.51±0.2°, 10.16±0.2°, 14.54±0.2°, and 17.01±0.2°.

21. The triphenylacetate salt according to claim 19 or 20, wherein the crystal form II has an X-ray powder diffraction pattern comprising characteristic peaks at diffraction angles 20 of 4.83±0.2°, 5.61±0.2°, 6.85±0.2°, 8.51±0.2°, 10.16±0.2°, 14.54±0.2°, and 17.01±0.2°.

22. The triphenylacetate salt according to any one of claims 19-21, wherein the crystal form II has an X-ray powder diffraction pattern substantially consistent with FIG. 4.

23. The triphenylacetate salt according to any one of claims 19-22, wherein the method for preparing the crystal form II comprises crystallizing the salt of formula (I-I) in MTBE solvent to give the crystal form II.

24. The triphenylacetate salt according to any one of claims 19-23, wherein the crystal form II has a differential scanning calorimetry profile comprising endothermic peaks at 61.9 °C, 146.8 °C, and 184.6 °C.

25. The triphenylacetate salt according to any one of claims 19-24, wherein the crystal form II has a differential scanning calorimetry profile substantially consistent with FIG. 5B.

26. The triphenylacetate salt according to any one of claims 1-11, wherein the salt is a crystal, and the crystal is crystal form III formed by crystallizing the salt of formula (I-I): 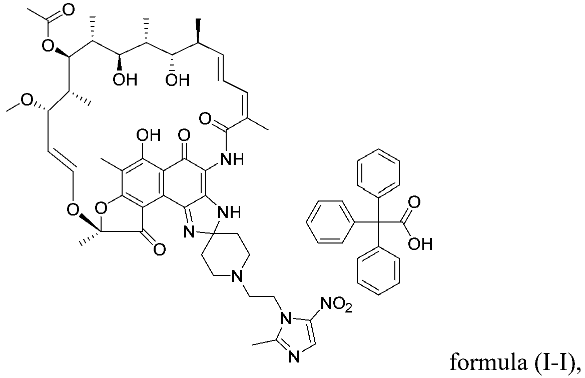 the crystal form III has an X-ray powder diffraction pattern comprising characteristic peaks at diffraction angles 20 of 9.57±0.2°, 11.49±0.2°, 12.14±0.2°, 16.18±0.2°, 18.26±0.2°, 19.61±0.2°, and 19.92±0.2°.

27. The triphenylacetate salt according to claim 26, wherein the crystal form III has an X-ray powder diffraction pattern comprising characteristic peaks at one or more diffraction angles 20 of 4.10±0.2°, 5.00±0.2°, 5.76±0.2°, 19.15±0.2°, 20.81±0.2°, 21.60±0.2°, and 24.52±0.2°.

28. The triphenylacetate salt according to claim 26 or 27, wherein the crystal form III has an X-ray powder diffraction pattern comprising characteristic peaks at diffraction angles 2θ of 4.10±0.2°, 5.00±0.2°, 5.76±0.2°, 19.15±0.2°, 20.81±0.2°, 21.60±0.2°, and 24.52±0.2°.

29. The triphenylacetate salt according to any one of claims 26-28, wherein the crystal form III has an X-ray powder diffraction pattern substantially consistent with FIG. 6.

30. The triphenylacetate salt according to any one of claims 26-29, wherein the method for preparing the crystal form III comprises crystallizing the salt of formula (I-I) in a solvent mixture of 2-methyltetrahydrofuran and n-heptane to give the crystal form III.

31. The triphenylacetate salt according to any one of claims 26-30, wherein the crystal form III has a differential scanning calorimetry profile comprising endothermic peaks at 64.7 °C, 142.8 °C, 159.4 °C, and 177.0 °C.

32. The triphenylacetate salt according to any one of claims 26-31, wherein the crystal form III has a differential scanning calorimetry profile substantially consistent with FIG. 7B.

33. The triphenylacetate salt according to any one of claims 1-11, wherein the salt is a crystal, and the crystal is crystal form IV formed by crystallizing the salt of formula (I-I): 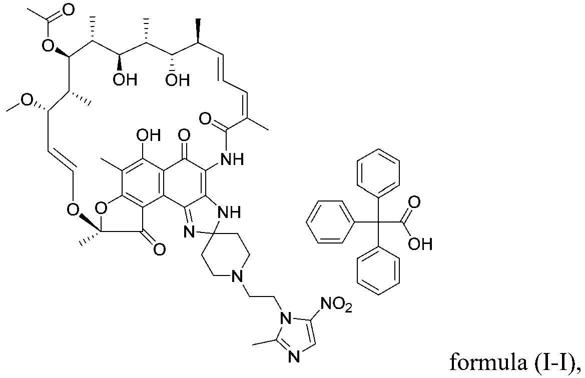 the crystal form IV has an X-ray powder diffraction pattern comprising characteristic peaks at diffraction angles 20 of 10.64±0.2°, 11.54±0.2°, 14.64±0.2°, 16.06±0.2°, 18.08±0.2°, and 23.89±0.2°.

34. The triphenylacetate salt according to claim 33, wherein the crystal form IV has an X-ray powder diffraction pattern comprising characteristic peaks at one or more diffraction angles 20 of 4.85±0.2°, 7.21±0.2°, 18.82±0.2°, 20.09±0.2°, 24.26±0.2°, and 30.24±0.2°.

35. The triphenylacetate salt according to claim 33 or 34, wherein the crystal form IV has an X-ray powder diffraction pattern comprising characteristic peaks at diffraction angles 20 of 4.85±0.2°, 7.21±0.2°, 18.82±0.2°, 20.09±0.2°, 24.26±0.2°, and 30.24±0.2°.

36. The triphenylacetate salt according to any one of claims 33-35, wherein the crystal form IV has an X-ray powder diffraction pattern substantially consistent with FIG. 8.

37. The triphenylacetate salt according to any one of claims 33-36, wherein the method for preparing the crystal form IV comprises heating the crystal form according to any one of claims 26-32 to 160 °C to form the crystal form IV.

38. The triphenylacetate salt according to any one of claims 33-37, wherein the crystal form IV has a differential scanning calorimetry profile comprising an endothermic peak at 182.8 °C.

39. The triphenylacetate salt according to any one of claims 33-38, wherein the crystal form IV has a differential scanning calorimetry profile substantially consistent with FIG. 9B.

40. The triphenylacetate salt according to any one of claims 1-11, wherein the salt is a crystal, and the crystal is crystal form V formed by crystallizing the salt of formula (I-II): 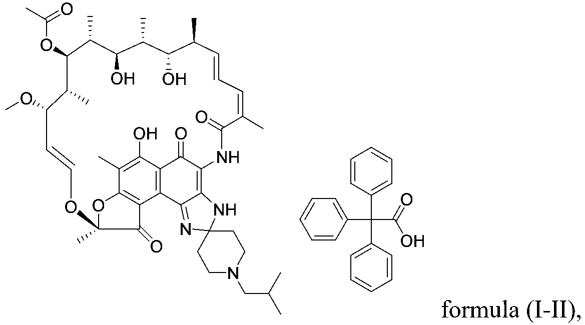 the crystal form V has an X-ray powder diffraction pattern comprising characteristic peaks at diffraction angles 20 of 7.65±0.2°, 10.84±0.2°, 11.71±0.2°, 12.26±0.2°, 20.60±0.2°, and 24.83±0.2°.

41. The triphenylacetate salt according to claim 40, wherein the crystal form V has an X-ray powder diffraction pattern comprising characteristic peaks at one or more diffraction angles 20 of 6.45±0.2°, 16.44±0.2°, 18.18±0.2°, 18.47±0.2°, and 26.54±0.2°.

42. The triphenylacetate salt according to claim 40 or 41, wherein the crystal form V has an X-ray powder diffraction pattern comprising characteristic peaks at diffraction angles 20 of 6.45±0.2°, 16.44±0.2°, 18.18±0.2°, 18.47±0.2°, and 26.54±0.2°.

43. The triphenylacetate salt according to any one of claims 40-42, wherein the crystal form V has an X-ray powder diffraction pattern substantially consistent with FIG. 14.

44. The triphenylacetate salt according to any one of claims 40-43, wherein the method for preparing the crystal form V comprises crystallizing the triphenylacetate salt of formula (I-II) in ethyl acetate solvent to give the crystal form V.

45. The triphenylacetate salt according to any one of claims 40-44, wherein the crystal form V has a differential scanning calorimetry profile comprising an endothermic peak at 164.0 °C.

46. The triphenylacetate salt according to any one of claims 40-45, wherein the crystal form V has a differential scanning calorimetry profile substantially consistent with FIG. 15B.

47. The triphenylacetate salt according to any one of claims 1-11, wherein the salt is a crystal, and the crystal is crystal form VI formed by crystallizing the salt of formula (I-II): 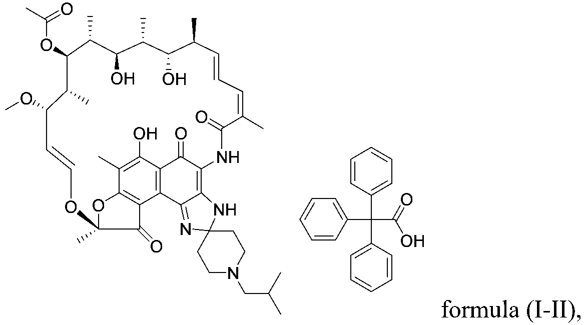 the crystal form VI has an X-ray powder diffraction pattern comprising characteristic peaks at diffraction angles 20 of 11.05±0.2°, 11.76±0.2°, 12.45±0.2°, 16.45±0.2°, 18.15±0.2°, 20.59±0.2°, and 24.77±0.2°.

48. The triphenylacetate salt according to claim 47, wherein the crystal form VI has an X-ray powder diffraction pattern comprising characteristic peaks at one or more diffraction angles 20 of 5.89±0.2°, 6.63±0.2°, 8.87±0.2°, 9.31±0.2°, 17.87±0.2°, 20.00±0.2°, and 24.26±0.2°.

49. The triphenylacetate salt according to claim 47 or 48, wherein the crystal form VI has an X-ray powder diffraction pattern comprising characteristic peaks at diffraction angles 20 of 5.89±0.2°, 6.63±0.2°, 8.87±0.2', 9.31±0.2°, 17.87±0.2°, 20.00±0.2°, and 24.26±0.2°.

50. The triphenylacetate salt according to any one of claims 47-49, wherein the crystal form VI has an X-ray powder diffraction pattern substantially consistent with FIG. 16.

51. The triphenylacetate salt according to any one of claims 47-50, wherein the method for preparing the crystal form VI comprises crystallizing the triphenylacetate salt of formula (I-II) in methyl tert-butyl ether solvent to give the crystal form VI.

52. The triphenylacetate salt according to any one of claims 47-51, wherein the crystal form VI has a differential scanning calorimetry profile comprising endothermic peaks at 127.2 °C, 140.6 °C, and 161.8 °C.

53. The triphenylacetate salt according to any one of claims 47-52, wherein the crystal form VI has a differential scanning calorimetry profile substantially consistent with FIG. 17B.

54. A method for preparing a salt, comprising: reacting a free base of the compound of formula (I) with triphenylacetic acid to form the salt according to any one of claims 1-53.

55. The method according to claim 54, comprising: reacting the free base of the compound of formula (I) with triphenylacetic acid in a solvent or a combination consisting of solvents selected from: (1) ethyl acetate, (2) 2-methyltetrahydrofuran and isopropyl acetate, (3) 2-methyltetrahydrofuran and n-heptane, (4) 2-butanone and n-heptane, (5) methyl tert-butyl ether, (6) methyl tert-butyl ether and n-heptane, (7) toluene, (8) isopropanol and n-heptane, (9) isopropyl acetate, (10) acetone, (11) methanol and methyl tert-butyl ether, and (12) n-heptane.

56. The method according to claim 54 or 55, comprising: mixing the free base of the compound of formula (I), ethyl acetate, and triphenylacetic acid sequentially to give a reaction mixture.

57. The method according to claim 56, comprising: incubating the reaction mixture at a reaction temperature of 20-40 °C.

58. The method according to claim 54 or 55, comprising: mixing triphenylacetic acid, 2-methyltetrahydrofuran, the free base of the compound of formula (I), and isopropyl acetate sequentially to give a reaction mixture.

59. The method according to claim 54 or 55, comprising: mixing triphenylacetic acid, 2-methyltetrahydrofuran, the free base of the compound of formula (I), and n-heptane sequentially to give a reaction mixture.

60. The method according to claim 58 or 59, comprising: incubating the reaction mixture at a reaction temperature of 50-75 °C.

61. The method according to claim 54 or 55, comprising: mixing the free base of the compound of formula (I), 2-butanone, triphenylacetic acid, and n-heptane sequentially to give a reaction mixture.

62. The method according to claim 61, comprising: incubating the reaction mixture at a reaction temperature of 40-60 °C.

63. The method according to any one of claims 54-62, wherein the salt formed from the free base of the compound of formula (I) and triphenylacetic acid is a crystal, and the method further comprises recrystallizing the crystal in a proper solvent to increase the purity.

64. The method according to claim 63, wherein the proper solvent comprises 2-butanone and n-heptane, or comprises acetone and n-heptane.

65. A method of preparing a medicament, comprising: providing the triphenylacetate salt according to any one of claims 1-53.

66. The method according to claim 65, comprising: 1) dissolving the triphenylacetate salt in a proper organic solvent to convert the salt into a free base, 2) adjusting the free base to a proper concentration using a proper organic solvent, and 3) using an organic solvent capable of precipitating a purified drug solid from a solution of the free base to give the drug.

67. The method according to claim 66, wherein the proper organic solvents in steps 1) and 2) are each independently selected from: dichloromethane, ethyl acetate, isopropyl acetate, methyl tert-butyl ether, and 2-methyltetrahydrofuran, and the organic solvents used in steps 1) and 2) have different solubilities.

68. The method according to claim 66, wherein the solvent capable of precipitating the purified drug solid is n-heptane.

69. The method according to any one of claims 65-68, comprising: 1) dissolving the triphenylacetate salt according to any one of claims 1-53 in a proper organic solvent, washing one or more times with a washing solution, collecting the washed organic phase, washing the washed organic phase with water, and collecting the water-washed organic phase; 2) adding a proper organic solvent having a solubility different from that of the organic solvent in step 1) to the collected water-washed organic phase in step 1) and concentrating at reduced pressure, and repeating the procedure until a free base concentrate of the compound of formula (I) is acquired; and 3) adding the free base concentrate in step 2) to an organic solvent capable of precipitating the purified drug solid in a nitrogen atmosphere, filtering, washing, and drying to give the drug.

70. The method according to claim 69, wherein the washing solution is selected from a proper weak base solution.

71. The method according to claim 69 or 70, wherein the washing solution is a sodium bicarbonate solution, a sodium carbonate solution, a potassium bicarbonate solution, or a potassium carbonate solution.

72. The method according to any one of claims 69-71, wherein the residue of the proper organic solvent in step 1) in the free base concentrate is equal to or less than 10.0%.

73. The method according to any one of claims 69-72, wherein step 3) comprises dropwise adding the free base concentrate into n-heptane with the internal temperature kept at 10-20 °C while stirring over a period of 3-8 h, and continuously stirring for 1-2 h after the dropwise addition.

74. A pharmaceutical composition, comprising: the triphenylacetate salt according to any one of claims 1-53, and optionally a pharmaceutically acceptable carrier.

75. The pharmaceutical composition according to claim 74, comprising: a second therapeutic agent.

76. The pharmaceutical composition according to claim 75, wherein the second therapeutic agent is selected from: a proton pump inhibitor, an acid inhibitor, an efflux pump inhibitor, an anesthetic, an antifungal agent, an antiviral agent, an antibacterial agent, an antiprotozoal agent, an anti-inflammatory agent, an anticoagulant, a platelet aggregation inhibitor, an antipyretic, a lipid-lowering agent, and a zinc salt.

77. A kit, comprising the triphenylacetate salt according to any one of claims 1-53 and/or the pharmaceutical composition according to any one of claims 74-76.

78. The kit according to claim 77, comprising written instructions and/or machine-readable electronic instructions.

79. A method for inhibiting or preventing bacterial growth, comprising: administering an effective amount of the triphenylacetate salt according to any one of claims 1-53, the pharmaceutical composition according to any one of claims 74-76, and/or the kit according to claim 77 or 78.

80. A method for treating a disease, comprising: administering to a patient in need an effective amount of the triphenylacetate salt according to any one of claims 1-53, the pharmaceutical composition according to any one of claims 74-76, and/or the kit according to claim 77 or 78.

81. The method according to claim 80, wherein the patient is infected with a bacterium.

82. The method according to claim 79 or 81, wherein the bacterium is selected from: Helicobacter pylori, Mycobacterium tuberculosis, Nontuberculous mycobacteria, Acinetobacter baumannii, Bacteroides fragilis, Bifidobacterium longum, Ruminococcus spp., Prevotella spp., Clostridium perfringens, Clostridium difficile, Lactobacillus acidophilus, Eggertella lenta, Fusobacterium nucleatum, Gardnerella vaginalis, Mobiluncus mulieris, Peptostreptococcus spp., Porphyromonas asaccharolyticus, Prevotella bivia, Propionibacterium acnes, and Veillonella parvula.

83. Use of the triphenylacetate salt according to any one of claims 1-53, the pharmaceutical composition according to any one of claims 74-76, and/or the kit according to claim 77 or 78 in inhibiting or preventing bacterial growth.

84. Use of the triphenylacetate salt according to any one of claims 1-53, the pharmaceutical composition according to any one of claims 74-76, and/or the kit according to claim 77 or 78 in preparing a medicament for treating and/or preventing a disease or condition caused by bacterial infection.

85. The use according to claim 83 or 84, wherein the bacterium is selected from: Helicobacter pylori, Mycobacterium tuberculosis, Nontuberculous mycobacteria, Acinetobacter baumannii, Bacteroides fragilis, Bifidobacterium longum, Ruminococcus spp., Prevotella spp., Clostridium perfringens, Clostridium difficile, Lactobacillus acidophilus, Eggertella lenta, Fusobacterium nucleatum, Gardnerella vaginalis, Mobiluncus mulieris, Peptostreptococcus spp., Porphyromonas asaccharolyticus, Prevotella bivia, Propionibacterium acnes, and Veillonella parvula.

86. The use according to claim 84, wherein the disease or condition is selected from: gastritis, gastric ulcer, bacterial vaginosis, diarrhea, pneumonia, appendicitis, cholecystitis, otitis media, endocarditis, endometritis, brain abscess, myocardial necrosis, osteomyelitis, peritonitis, empyema, salpingitis, septic arthritis, liver abscess, sinusitis, pelvic inflammatory disease, and bacteremia; and upper respiratory tract infection, lower respiratory tract infection, skin and soft tissue infection, bone and joint infection, lung infection, intra-abdominal infection, eye infection, ear infection, oral infection, and surgical infection.
